# EUROPÄISCHE PATENTSCHRIFT

(11) **EP 2 714 653 B1**
(45) Veröffentlichungstag und Bekanntmachung des Hinweises auf die Patenterteilung: **03.05.2017**
(21) Anmeldenummer: 12723194.2
(22) Anmeldetag: 24.05.2012
(51) Int. Cl.: C07C 309/00, C07C 301/00, C09J 4/00, A61K 6/083, C07F 7/18, C08G 77/392, C08G 77/28, C09J 183/08

(54) **VERBINDUNGEN MIT (METH)ACRYLAT-RESTEN UND SULFONAT- ODER SULFATGRUPPEN, POLYMERE UND KONDENSATE DARAUS SOWIE VERWENDUNG DER POLYMERE UND KONDENSATE**
COMPOUNDS CONTAINING (METH)ACRYLATE GROUPS AND SULFONATE OR SULFATE GROUPS, POLYMERS AND CONDENSATES MADE THEREFROM AND USE OF THE POLYMERS AND CONDENSATES
COMPOSÉS COMPRENANT DES RADICAUX (MÉTH)ACRYLATE ET DES GROUPES SULFONATE OU SULFATE, POLYMÈRES ET CONDENSATS À PARTIR DE CEUX-CI ET UTILISATION DES POLYMÈRES ET DES CONDENSATS

(30) Priorität: 27.05.2011 DE 102011050672; 27.05.2011 EP 11167853
(43) Veröffentlichungstag der Anmeldung: 09.04.2014
(73) Patentinhaber: Fraunhofer Gesellschaft zur Förderung der angewandten Forschung E.V., 80686 München (DE)
(72) Erfinder: WOLTER, Herbert, 97941 Tauberbischofsheim (DE); SEYFRIED, Mona, 97070 Würzburg (DE); NIQUE, Somchith, 97249 Eisingen (DE)
(74) Vertreter: Strehl Schübel-Hopf & Partner
(86) Internationale Anmeldenummer: PCT/EP2012/059671
(87) Internationale Veröffentlichungsnummer: WO 2012/163781

(56) Entgegenhaltungen:
- EP-A1- 1 767 523
- WO-A1-2006/122074
- WO-A1-2012/019704
- DE-A1- 10 206 451
- DE-T2- 60 216 930
- US-A- 3 748 132
- ROBERT L. KERR ET AL: "New Type of Li Ion Conductor with 3D Interconnected Nanopores via Polymerization of a Liquid Organic Electrolyte-Filled Lyotropic Liquid-Crystal Assembly", JOURNAL OF THE AMERICAN CHEMICAL SOCIETY, Bd. 131, Nr. 44, 11. November 2009 (2009-11-11), Seiten 15972-15973, XP55030501, ISSN: 0002-7863, DOI: 10.1021/ja905208f
- YANJIE XU ET AL: "Heterogeneous Catalysis Using a Nanostructured Solid Acid Resin Based on Lyotropic Liquid Crystals", JOURNAL OF THE AMERICAN CHEMICAL SOCIETY, Bd. 126, Nr. 6, 1. Februar 2004 (2004-02-01), Seiten 1616-1617, XP55030505, ISSN: 0002-7863, DOI: 10.1021/ja038501i
- SIEBURTH SM, LANG J.: "A PRACTICAL SYNTHESIS OF DIFUNCTIONAL ORGANOSILANE REAGENTS AND THEIR APPLICATION TO THE DIELS-ALDER REACTION", J. ORG. CHEM., Bd. 64, 1999, Seiten 1780-1781, XP002685036,

## Beschreibung

Die vorliegende Erfindung betrifft Verbindungen mit mindestens einem (Meth)Acryl-Rest, mindestens einer Sulfonat- oder Sulfatgruppe sowie einer weiteren, meist reaktiven Gruppe, deren Polymerisationsprodukte sowie die Verwendung der Monomeren und der Polymeren im medizinischen Bereich, insbesondere in der Zahnmedizin. Soweit die Erfindung SilanVerbindungen betrifft, umfasst sie auch die daraus erhältlichen Kondensate sowie deren Verwendung.

Polymerisierbare, organische Verbindungen mit Säuregruppen sind für medizinische Produkte zum Erreichen gewünschter Materialeigenschaften wie Benetzung, Ätzwirkung, Komplexierung und damit Haftung auf biologischen Grenzflächen wichtige Bestandteile. Dentaladhäsive basieren auf solchen konventionellen, monomeren Verbindungen, weisen allerdings noch einige erhebliche Defizite auf. Ein wesentliches Problem dabei ist, das die Ätzwirkung im Rahmen der Self-etch-Applikation oftmals nicht ausreicht, um die notwendigen retentiven Strukturen zu realisieren, die für die Haftung und damit einen langlebigen Verbund zwischen Zahngewebe und Restaurationsmaterial erforderlich sind. Somit ist oftmals ein vorheriger separater Ätzschritt mit einem Ätzgel nicht vermeidbar, was wiederum die Fehleranfälligkeit und die Behandlungskosten erhöht. Hinsichtlich der zunehmenden Anforderungen hinsichtlich Biokompatibilität (verwiesen sei auf die Allergiediskussion in Verbindung mit dentalen Monomeren) bieten obige Systeme ebenfalls keine Lösung an. Da die Komponenten des Adhäsivs bei einer Restauration den Zahnwurzeln sowie Blutgefäßen am nächsten kommen, ist es aus toxikologischer Sicht von besonderem Interesse, monomerfreie Systeme bereitzustellen.

In der Patentanmeldung DE 44 16 857 C1 werden Carbonsäure-funktionalisierte (Meth)acrylatalkoxysilane beschrieben. Sie zeichnen sich durch eine Vielfalt von Möglichkeiten aus, die Eigenschaften der daraus resultierenden anorganisch-organischen Verbundpolymere zu variieren bzw. einzustellen. Bedingt durch die enthaltenen Carbonsäuregruppen ergeben sich zusätzliche Reaktionsmöglichkeiten (z. B. Glasionomerreaktionen) sowie eine verbesserte Haftung auf anorganischen Oberflächen. Die Ätzwirkung (s. Self-etch-Applikation) einer Carbonsäuregruppe ist aber bei weitem nicht so stark, wie es manchmal wünschenswert wäre. Entsprechendes gilt für die in EP 1 377 628 B1 beschriebenen phosphonsäurebasierten Systeme. Mit hybridpolymerbasierten Systemen kann im Rahmen der wünschenswerten Self-etch-Applikationen daher bisher kein ausreichend stabiler Verbund zwischen Zahngewebe und Restaurationsmaterial erzielt werden.

Für eine Reihe von Anwendungszwecken wie die Stabilisierung wässriger Silikate oder die Herstellung von elektroviskosen Flüssigkeiten, Emulgatoren, Detergentien oder Schaumbildnern wurden monomere oder kondensierte Silane entwickelt, die Sulfonat- oder Sulfatgruppen enthalten. So beschreibt US 6,777,521 Siliconsulfat-Polymere, die durch die Umsetzung entsprechender Epoxyverbindungen mit Metallsulfat erhältlich sind. Aus US 3,328,449 sind sulfopropylierte organofunktionelle Silane und Siloxane bekannt, die mit Hilfe der Umsetzung von Sultonen erhalten werden können. US 4,777,277 offenbart Organosiloxansulfosuccinate, in denen ein sulfonierter Bernsteinsäureester mit dem Sauerstoffatom der Estergruppe über eine Alkylengruppe an ein Siliciumatom angebunden ist. US 4,503,242 zeigt in Beispiel 1 die Herstellung eines hydrolytisch kondensierbaren Bis-sulfosuccinatamids eines Diaminosilans, erhalten durch die Umsetzung der freien Carbonsäure des entsprechenden Succinatamids mit Natriumsulfit. Ein Silan, das eine Sulfonatgruppe und eine Hydroxygruppe an einem Alkylenoxyalkylenrest des Siliciums trägt, ist in US 5,427,706 offenbart.

Die Verwendung von rein organischen Monomeren, die sowohl eine terminale Sulfonatgruppe als auch eine ungesättigte olefinische Gruppe tragen, für das gleichzeitige Ätzen und Grundieren ("priming") von Zähnen schlägt US 2002/0119426 A1 vor. Das im Beispiel verwendete AMPS (2-Acrylamido-2-methylpropansulfonsäure) ist ein kommerziell erhältliches Produkt. Auch US 6,759,449 B2 offenbart Dentalkleber-Zusammensetzungen, die sowohl eine organisch polymerisierbare (Meth)Acrylsäure- als auch eine saure Gruppe tragen. Dabei wird zwischen Sulfonatgruppen und Phosphonat- oder anderen sauren Gruppen hinsichtlich der Verwendbarkeit der Verbindungen und ihrer Eigenschaften nicht unterschieden. Gleiches gilt für US 2003/0055124 A1; nur für darin gezeigte (Meth)Acrylamido-phosphonsäuren, nicht aber für ebenfalls aufgezeichnete, entsprechende Sulfonsäuren werden Angaben zur Herstellung gemacht. Eine weitere Anmeldung im Wesentlichen derselben Erfindergruppe ,
US 2008/0194730, schlägt wiederum die Verwendung von selbstätzenden polymerisierbaren N-substituierten (Meth)Acrylsäureamid-Monomeren für Dentalkomposite vor, die zusätzlich eine saure Einheit tragen, ausgewählt unter Phosphonsäure- und Sulfonsäure-Einheiten. N-Methacryloylaminoalkylsulfonsäuren können nach den Erläuterungen der EP 1 421 927 A1 als selbstätzende Primer für dentale Zwecke eingesetzt werden.

Dentalkleber-Zusammensetzungen aus sauer polymerisierbaren Nanoteilen in einer wässrigen Phase offenbart DE 102 06 451 A1. Die Nanoteilchen bestehen aus Siloxanen, an denen sowohl saure als auch organisch polymerisierbare Gruppen gebunden vorliegen. Die sauren Gruppen können entweder Phosphonat- oder Sulfonatgruppen sein; individuelle spezifische Vorteile für die eine oder die andere Gruppe werden nicht genannt. Das einzige Anwendungsbeispiel nennt einen spezifischen Haftungswert eines Dentalklebstoffs aus einem phosphonsäurehaltigen Material an einer Zahnoberfläche. Ein Verfahren zum Herstellen sulfonatgruppenhaltiger Silane ist weder allgemein noch hinsichtlich der gezeichneten Verbindungen angegeben.

Es besteht ein Bedarf an organisch polymerisierbaren Monomeren mit überlegenen Eigenschaften für die Anwendung insbesondere im Dentalbereich. Besonders relevant sind hier eine verbesserte Haftung und/oder eine verbesserte Ätzfunktion und/oder eine Anpassung der optischen Eigenschaften für das kosmetische Erscheinungsbild. Hier Abhilfe zu schaffen, ist die Aufgabe der vorliegenden Erfindung.

In Lösung der Aufgabe werden Verbindungen bereitgestellt, die mindestens drei Funktionalitäten aufweisen, nämlich (a) eine Sulfonsäure- oder Sulfonatgruppe der Formel -(O)_{d}-SO₃M mit d = 0 oder 1 und M = Wasserstoff oder ein einwertiges Metallkation oder der entsprechende Anteil eines mehrwertigen Metallkations für eine sehr gute Ätzwirkung, (b) einen (Meth)Acrylrest als organisch polymerisierbare Gruppe, durch die das Material nach dem Aufbringen auf oder in den Zahn ausgehärtet werden kann, sowie (c) entweder (c1) mindestens einen weiteren (Meth)Acrylrest oder eine anorganisch kondensierbare Gruppe, um eine besonders gute Vernetzung des Dentalmaterials zu ermöglichen, und/oder (c2) eine weitere Säurefunktion, um die Ätzwirkung des Moleküls zu verbessern, und/oder (c3) eine Funktion, durch die sich der Brechungsindex eines aus den Verbindungen hergestellten Materials erhöht, was zu einer verbesserten Transluzenz und damit einer verbesserten Anpassung an die Zahnfarbe führt. Bei der Säurefunktion gemäß (c2) kann es sich um eine zusätzliche Sulfonsäure- oder Schwefelsäuregruppe handeln; alternativ kann eine andere saure Gruppe vorhanden sein, beispielsweise ein Carbonsäuregruppe.

Verbindungen, die (a) eine Sulfonatgruppe, (b) eine Acrylgruppe oder eine Methacrylgruppe und (c) eine Silylgruppe enthalten, sind vorzugsweise dann von der Erfindung ausgeschlossen, wenn diese Verbindungen ein dreibindiges Stickstoffatom enthalten und jede der drei Gruppen (a), (b) und (c) an einem anderen Substituenten des Stickstoffatoms gebunden vorliegt, sofern die (Meth)Acrylgruppe als (Meth)Acrylamid oder die Acrylgruppe über die als Substituent am C2 befindliche Methylgruppe an das Stickstoffatom gekoppelt ist, und zwar insbesondere dann, wenn das Schwefelatom der Sulfonatgruppe durch drei oder vier Kohlenstoffatome vom Stickstoffatom getrennt ist. Vor allem sollen die drei folgenden Verbindungen gegebenenfalls von der Erfindung nicht umfasst sein, die in DE 102 06 451 A1 und US 7041709 dargestellt sind, ohne dass diese Druckschriften eine Möglichkeit aufzeigen würden, sie herzustellen: (RO)₃Si(CH₂)₃N[(CH₂)ₓSO₃Na][C(O)C(CH₃)=CH₂] mit x = 3 und x = 4 und (RO)₃Si(CH₂)₃N[(CH₂)₃SO₃Na][CH₂C(=CH₂)C(O)OR], worin die Substituenten R jeweils unabhängig voneinander Wasserstoff, eine substituierte oder unsubstituierte Alkylgruppe mit 1 bis 20 Kohlenstoffatomen, eine substituierte oder unsubstituierte, ggf. einen Cyclus enthaltende Alkylen- bzw. Alkenylgruppe mit 2 bis 20 Kohlenstoffatomen, eine substituierte oder unsubstituierte Alkylarylgruppe, eine substituierte oder unsubstituierte Arylen- bzw. Arylgruppe oder eine substituierte oder eine unsubstituierte Heteroarylen- bzw. Heteroarylgruppe mit jeweils 5 bis 20 Kohlenstoffatomen ist.

Den Erfindern ist es gelungen, Wege zur Herstellung von Verbindungen aufzufinden, die sowohl organisch polymerisierbare Gruppen, insbesondere (Meth)Acrylgruppen, als auch Sulfat- oder Sulfonatgruppen aufweisen und die gekennzeichnet sind durch mindestens eine weitere Gruppe, ausgewählt unter einem weiteren (Meth)Acrylrest, einem anorganisch kondensierbaren Silylrest und einer weiteren Säurefunktion. Anstelle der weiteren Gruppe - oder aber auch zusätzlich - können die Verbindungen eine Thioethergruppe aufweisen, durch die sich der Brechungsindex von aus den Verbindungen hergestellten Dentalmaterialien erhöht.

In einer ersten Ausführungsform der Erfindung sind die erfindungsgemäßen Verbindungen Silane, die sich mit der nachstehenden Formel (I) darstellen lassen:

R⁷ₐR²_{b}SiZ_{4-a-b} (I)

worin R⁷ ein hydrolytisch (d.h. unter Hydrolysebedingungen) kondensierbarer Rest ist, R² substituiertes oder unsubstituiertes, geradkettiges, verzweigtes oder mindestens einen Cyclus aufweisendes Alkyl, Aryl, Arylalkyl, Alkylaryl oder Alkylarylalkyl ist, ausnahmsweise aber stattdessen ein entsprechendes Alkenyl sein bzw. ein solches umfassen kann, dessen Kohlenstoffkette in allen Fällen gegebenenfalls durch -O-, -S-, -NH-, -S(O)-, -C(O)NH-, -NHC(O)-, -C(O)O- -C(O)S, -NHC(O)NH- oder C(O)NHC(O)-Gruppen unterbrochen sein kann, die ggf. in beide möglichen Richtungen weisen können, Z ein Rest ist, in welchem mindestens eine (Meth)Acrylgruppe und mindestens entweder eine Sulfonat- oder eine Sulfatgruppe unmittelbar oder mittelbar über eine unsubstituierte oder substituierte Kohlenwasserstoffgruppe am Siliciumatom gebunden vorliegen, a 1, 2 oder 3 ist, b 0, 1 oder 2 ist und a+b zusammen 1, 2 oder 3 sind.

Bedingt durch die erfindungsgemäß vorgeschlagenen Herstellungsverfahren werden solche Silane der Formel (I) bevorzugt, in denen dann, wenn eine Sulfonatgruppe über eine durch ein oder mehrere Stickstoffatome unterbrochene Alkylengruppe an das Siliciumatom gebunden ist, entweder nicht mehr als zwei Kohlenstoffatome zwischen der Sulfonatgruppe und dem nächstgelegenen Stickstoffatom vorhanden sind oder aber diese Alkylengruppe durch mindestens eine -O-, -S-, -NH-, -C(O)NH-, -NHC(O)-, -S(O)- oder -C(O)O- Gruppen unterbrochen ist.

Die Strukturen dieser neuartigen Silane sind in den beiden nachfolgenden Schemazeichnungen abstrahiert dargestellt. Der Alkoxysilanteil (k-fach vorhanden, d.h. es kann mehr als ein Silylrest im Molekül vorhanden sein, was z.B. im Falle einer Verbindung gegeben sein kann, in der zwei oder mehr (Meth)Acrylsäurereste organisch miteinander verknüpft und in der angegebenen Weise an jeweils ein Si-Atom gebunden sind) kann wie üblich im Rahmen des allgemein für die Herstellung von Kieselsäure(poly)kondensaten verwendeten Sol-Gel Prozesses zum Aufbau der anorganischen Oligomer- oder Polymerstruktur eingesetzt werden. Diese kann wie aus dem Stand der Technik bekannt auch vorliegend in Abhängigkeit von der Anzahl der hydrolytisch kondensierbaren Reste und den jeweiligen Hydrolysebedingungen die Form von Dimeren, Ketten, Ringen, Leitern (d.h. zweidimensionalen Flächen) oder dreidimensionalen Raumstrukturen besitzen. Der organisch polymerisierbare Molekülteil, bestehend aus einer variablen Anzahl m von Doppelbindungen in R'₆, die mit dem Silanteil verknüpft sind, kann zum Aufbau eines additiven organischen Netzwerks eingesetzt werden. Die Verbindungseinheit ist organischer Natur und variabel in Länge, Struktur und Zusammensetzung. Alle Molekülteile können zur Eigenschaftsmodifikation genutzt werden.

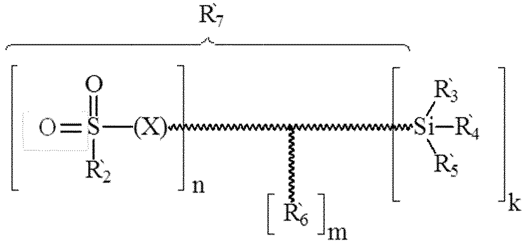

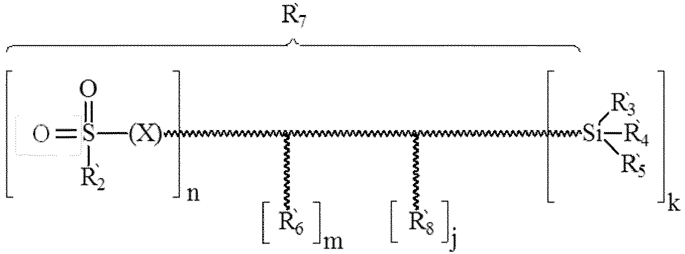

Hierbei haben die Gruppen und Indices die folgende Bedeutung:
R'₂ steht für OH oder ein Salz -OM mit M = einwertiges Metallkation oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium,
X bedeutet Sauerstoff oder ist nicht vorhanden,
R'₃, R'₄, R'₅ sind entweder Reste, die unter Ausbildung von Si-O-Si-Brücken einer hydrolytischen Kondensation unterworfen werden können wie OH, -OR, Halogen, z.B. Cl, oder Reste, die über ein Kohlenstoffatom an das Siliciumatom gebunden sind; im einfachsten Falle handelt es sich bei letzterem um Methyl,
R'₇ bezeichnet den organischen Rest, der sowohl die Sulf(on)atgruppe als auch den doppelbindungshaltigen Rest aufweist; dieser ist über ein Kohlenstoffatom an das Silicium gebunden.
R'₆ ist ein doppelbindungshaltiger Rest, in der Regel ein (Meth)Acrylrest.
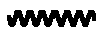 bezeichnet einen organischen Rest, der aufgrund der gewählten Reaktionsführungen in der Regel mindestens durch eine Verknüpfungsgruppe oder ein Sauerstoffatom, ein Schwefelatom oder eine sekundäre oder tertiäre Aminogruppe unterbrochen ist. Als Verknüpfungsgruppen dienen vorliegend vor allem -C(O)O- und -C(O)NH-Gruppen, stattdessen können aber auch -NHC(O)-, -NHC(O)O, -C(O)NHC(O), -NHC(O)NH- -S(O)- oder die entsprechenden schwefelhaltigen Gruppen als Verknüpfungen dienen.
R'₈ bedeutet -CO₂H oder OH.
n, m und j bedeuten jeweils mindestens 1, können aber in bestimmten Fällen 2 oder 3 sein oder sogar einen noch höheren Wert annehmen.

In einer zweiten Ausführungsform der Erfindung lassen sich die erfindungsgemäßen Verbindungen mit der nachstehenden Formel (II) darstellen: 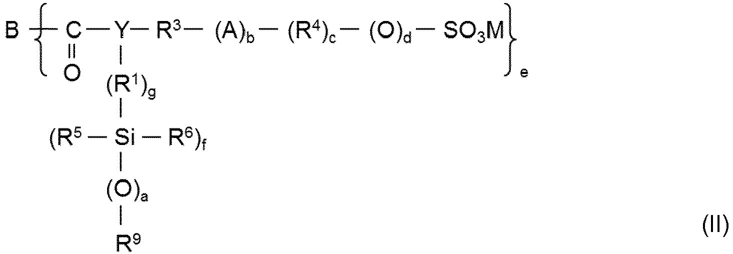 worin
R¹ ein zweibindiger Kohlenwasserstoffrest ist, der, sofern f = 1 ist, über ein Kohlenstoffatom am Siliciumatom gebunden ist,
R⁹ H oder Alkyl ist und zusätzlich im Falle von a = 0, f =1 und g =1
ein anderer hydrolytisch kondensierbarer Rest als Alkoxy oder 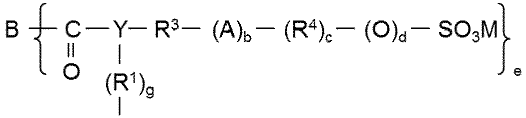 sein kann,
R³ ein unsubstituiertes oder mit einer funktionellen Gruppe substituiertes, geradkettiges, verzweigtes oder mindestens einen Cyclus aufweisendes Alkylen ist,
A eine Verknüpfungsgruppe darstellt,
R⁴ eine gegebenenfalls durch O, S, NH oder NR⁸ unterbrochene und/oder gegebenenfalls funktionell substituierte Kohlenwasserstoffgruppe, vorzugsweise ein solches Alkylen bedeutet, M Wasserstoff oder ein einwertiges Metallkation oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium ist,
R⁵ und R⁶ unabhängig voneinander entweder unter Hydrolysebedingungen kondensierbare Reste oder substituiertes oder unsubstituiertes, geradkettiges, verzweigtes oder mindestens einen Cyclus aufweisendes Alkyl, Aryl, Arylalkyl, Alkylaryl oder Alkylarylalkyl sind, ausnahmsweise stattdessen aber auch ein entsprechendes Alkenyl, Arylalkenyl oder Alkenylaryl sein können,
R⁸ Alkyl oder Alkenyl mit vorzugsweise 1 bis 6 bzw. 2 bis 6 Kohlenstoffatomen oder ein (Meth)Acrylrest ist,
B Vinyl, 2-Allyl oder, im Falle von e > 1, ein organischer Rest mit e Vinylgruppen ist, die jeweils an eine in der geschweiften Klammer befindliche Gruppe gebunden vorliegen,
Y ein Stickstoffatom, -O-CH=, -S-CH= oder -NH-CH= ist, wobei jeweils das Sauerstoffatom, das Schwefelatom bzw. die NH-Gruppe eine Bindung zur benachbarten C(O)-Gruppe aufweist,
a = 0 oder 1 ist,
b = 0 oder 1 ist,
c = 0 oder 1 ist,
d = 0 oder 1 ist, e = 1, 2 oder 3 ist
f = 0 oder 1 ist und
g = 0 oder 1 ist,
wobei dann, wenn f = 1 ist, a und g beide ≠ 0 sind und
wobei dann, wenn f = 0 ist, zumindest einer der Reste R³ oder R⁴ einen funktionellen Substituenten trägt, der einen (Meth)Acrylrest oder eine saure Gruppe aufweist oder R⁴ ein mindestens durch S unterbrochenes Alkylen bedeutet.

In einer spezifischen Ausgestaltung der zweiten Ausführungsform muss dann, wenn in der Formel (II) Y ein Stickstoffatom, b = 0, c = 0 und vorzugsweise auch d = 0 ist, der Rest R³ ein ggf. substituiertes Ethylen sein, sowie dann, wenn Y ein Stickstoffatom, b = 0, c = 1 und vorzugsweise auch d = 0 ist, der Rest R⁴ = ein durch O, S, NH oder NR⁸ unterbrochenes und gegebenenfalls funktionell substituiertes Alkylen sein.

Wenn sowohl f als auch g = 0 sind, ist a vorzugsweise ebenfalls 0.

In einer Reihe von bevorzugten Ausführungsformen lassen sich die erfindungsgemäßen Silane der Formeln (I) bzw. (II) mit der nachstehenden Formel (la) darstellen: 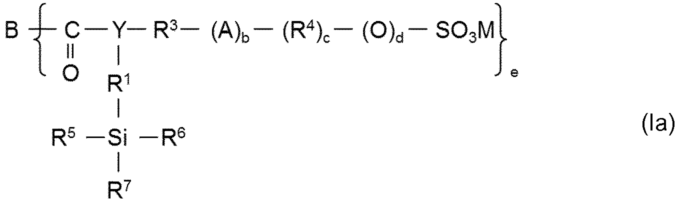 worin
R¹ eine über ein Kohlenstoffatom an das Siliciumatom gebundene Kohlenwasserstoffgruppe ist, wie sie weiter oben definiert wurde,
R⁷ ein hydrolytisch kondensierbarer Rest ist,
und die Reste R³, R⁴, R⁵, R⁶, B und Y sowie die Indices b, c, d, und e wie für Formel (II) definiert sind.

Auch in einer spezifischen Ausgestaltung der Formel (la) muss dann, wenn in der Formel (II) Y ein Stickstoffatom, b = 0, c = 0 und vorzugsweise auch d = 0 ist, der Rest R³ ein ggf. substituiertes Ethylen sein, sowie dann, wenn Y ein Stickstoffatom, b = 0, c = 1 und vorzugsweise auch d = 0 ist, der Rest R⁴ = ein durch O, S, NH oder NR⁸ unterbrochenes und gegebenenfalls funktionell substituiertes Alkylen sein.

Gemäß den obigen Formeln können sowohl der oder die Sulf(on)atreste als auch der oder die (Meth)Acrylreste unmittelbar oder mittelbar an der Kohlenwasserstoffgruppe gebunden sein, die mindestens aus R¹ besteht. In der Terminologie der Erfindung soll der Ausdruck "unmittelbar" dabei bedeuten, dass die genannten Reste ohne weitere Unterbrechungen der Kohlenstoffkette der Alkylgruppe durch S, O, NH oder eine Kupplungsgruppe am Silicium gebunden sind. "Mittelbar" bedeutet dementsprechend, dass diese Reste an Bestandteile des Moleküls angekoppelt sind, zwischen denen und der am Silicium gebundenen Alkylgruppe die genannten Atome bzw. Gruppen vorhanden sind, wie es z.B. für den Rest SO₃M in Formel la für den Fall ersichtlich ist, dass b = 1 ist oder dass c = 1 mit R⁴ gleich eine durch O, S oder, NH oder NR⁸ unterbrochene Gruppe ist.

Der zweibindige Kohlenwasserstoffrest R¹ kann in allen Ausführungsformen der Erfindung eine Alkylengruppe, eine Arylengruppe oder eine Gruppe sein, die sowohl Alkylen- als auch Aryleneinheiten aufweist. Die Alkylengruppe kann geradkettig oder verzweigt sein und/oder mindestens einen cyclischen Bestandteil besitzen. Die Gruppe kann dabei über ein Alkylen-Kohlenstoffatom oder über ein Arylen-Kohlenstoffatom am Siliciumatom gebunden sein. Sie ist unsubstituiert oder unsubstituiert, und sie kann durch ein oder mehrere Sauerstoffatome, Schwefelatome, Estergruppen, Aminogruppen oder Amidgruppen unterbrochen sein.

Die Verknüpfungsgruppe A in den Formeln (la) und (II) wird vorzugsweise ausgewählt unter (gelesen von links nach rechts in den Formeln la und II) C(O)NH, NHC(O), NR⁸C(O), C(O)O und OC(O), wobei R⁸ wie oben definiert ist. In Ausnahmefällen kann die Verknüpfungsgruppe A jedoch auch zusätzlich unter NHC(O)O, NR⁸C(O)O, NHC(O)NH, C(O)NHC(O) und -C(O)S-ausgewählt sein. Der Rest R⁴ in diesen Formeln ist in spezifischen Ausführungsformen substituiert mit mindestens einer Hydroxygruppe und/oder mit einem Rest R⁹COOM, worin R⁹ eine chemische Bindung oder ein C₁-C₆-Alkylenrest ist und M Wasserstoff oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium ist. Die genannten Varianten können unabhängig oder kombiniert verwirklicht sein.

Reste R⁷ in Formel (I) sowie Reste R⁵ und/oder R⁶ in den Formeln (la) und (II), die unter Hydrolysebedingungen kondensieren können, werden als anorganische Netzwerkbildner bezeichnet, da sich bei der hydrolytischen Kondensationsreaktion über sie ein Kieselsäurepolykondensat-Netzwerk ausbilden kann. Dem Fachmann ist entsprechend bekannt, mit welcher Anzahl dieser Reste welche Oligomer- bzw. Polymerstruktur erzielbar ist und welche Bedeutung sie annehmen können. Vorzugsweise sind R⁵ und R⁶ OH oder eine C₁-C₁₀-Alkoxygruppe, stärker bevorzugt eine C₁-C₄-Alkoxygruppe und ganz besonders bevorzugt Methoxy oder Ethoxy. R⁷ kann dieselbe Bedeutung besitzen wie R⁵ und/oder R⁶. R⁵, R⁶ und R⁷ können je nach Bedarf unabhängig voneinander aber auch ein Halogenid wie Cl, Wasserstoff, Acyloxy mit vorzugsweise 2 bis 5 Kohlenstoffatomen, Alkylcarbonyl mit vorzugsweise 2 bis 6 Kohlenstoffatomen oder Alkoxycarbonyl mit vorzugsweise 2 bis 6 Kohlenstoffatomen sein. In manchen Fällen können sie stattdessen die Bedeutung NR² mit R² gleich Wasserstoff, Alkyl mit vorzugsweise 1-4 Kohlenstoffatomen oder Aryl mit vorzugsweise 6-12 Kohlenstoffatomen haben.

Der Ausdruck "Sulf(on)at" umfasst die Sulfonat- und die Sulfatgruppe. Die Ausdrücke "Sulfonatgruppe" und "Sulfatgruppe" umfassen die jeweiligen Säuren und Salze.

Das Wort bzw. der Wortbestandteil "(Meth)Acryl..." soll die jeweiligen Methacryl- und Acrylverbindungen gleichermaßen umfassen. Die (Meth)Acrylreste können insbesondere Bestandteil eines (Meth)Acrylsäureesters, -thioesters oder -amids sein. (Meth)Acrylsäureamidreste sind gegenüber den sonstigen (Meth)Acrylresten wegen ihrer besseren Hydrolysebeständigkeit bevorzugt.

Ein Aspekt der Erfindung liegt darin, dass beim Aufbau der chemischen Strukturen der Verbindungen (II) ein bequemer Einbau der (Meth)Acrylgruppen dadurch erfolgen kann, dass diese in Form der freien bzw. einer aktivierten Säure umgesetzt werden. Dies hat zur Folge, dass die (Meth)Acrylgruppe als (Meth)Acrylsäureester, -amid oder-thioester in die Strukturen eingebaut vorliegt.

Mit wenigen Ausnahmen werden die Synthesen so geführt, dass für die Addition der Sulfonsäure- bzw. Sulfatgruppe an das bereits eine (Meth)Acrylgruppe enthaltende Molekül eine C=C-Doppelbindung zur Verfügung steht. Nach Wahl kann an diese entweder Natriumsulfit oder eine Sulfonsäure mit einem bequem addierbaren Rest wie eine Thio- oder Aminoalkansulfonsäure addiert werden. Alternativ kann die Anbindung der Sulfonsäuregruppe auch nach dem umgekehrten Prinzip erfolgen, indem ein Thio- oder Aminoalkylsilan bzw. eine entsprechende silanfreie Verbindung mit einer Alkylensulfonsäure zur Reaktion gebracht wird. Bei diesem Verfahren ist natürlich eine Kettenverlängerung durch die Kohlenstoffatome der Alkylengruppe unvermeidlich, weshalb die erstere Variante gegenüber der zweiten bevorzugt ist. Schließlich gibt es auch noch die Möglichkeit, die Ringöffnung eines reaktiven oder gespannten Heterorings, insbesondere eines Dreirings, mit Sulfit oder einer Hydroxy-, Thio- oder Aminoalkansulfonsäure zu bewirken. Diese Variante hat den Vorteil, dass bei der Ringöffnung eine weitere reaktive Gruppe entsteht, die für die folgende Anbindung der aktivierten (Meth)Acrylsäure genutzt werden kann. Der Heteroring kann alternativ auch mit Hilfe eines Sulfats geöffnet werden; in diesen Fällen erhält man ein sulfatgruppenhaltiges Produkt.

Wie oben erwähnt, betrifft eine Ausgestaltung der Erfindung Silane der Formeln (la) bzw. (II) mit f = 1 und g = 1. Bedingt durch die erfindungsgemäß vorgeschlagenen Herstellungsverfahren werden hier solche Silane bevorzugt, in denen dann, wenn eine Sulfonatgruppe über eine durch ein oder mehrere Stickstoffatome unterbrochene Alkylengruppe an das Siliciumatom bzw. an die (ggf. nächstgelegene) (Meth)Acrylgruppe gebunden ist, entweder nicht mehr als zwei Kohlenstoffatome zwischen der Sulfonatgruppe und dem nächstgelegenen Stickstoffatom vorhanden sind oder aber diese Alkylengruppe durch mindestens eine -O-, -S-, -NH-, -C(O)NH-, -NHC(O)- oder -C(O)O-Gruppen unterbrochen ist.

Insgesamt stehen erfindungsgemäß vier grundlegende Varianten für die Herstellung von Silanen mit der Formel (I), (la) bzw. (II) mit f = 1 zur Verfügung wie folgt:

### Variante (A):

(a) ein Silan mit einer über ein Kohlenstoffatom an das Si-Atom gebundenen Kohlenwasserstoffgruppe wird bereitgestellt, die mindestens zwei funktionelle Gruppen trägt, ausgewählt unter primären Aminen, sekundären Aminen, Hydroxygruppen und Thiolgruppen,
(b) eine erste der beiden funktionellen Gruppen wird mit gegebenenfalls aktivierter (Meth)Acrylsäure und die zweite der beiden funktionellen Gruppen wird mit einer gegebenenfalls aktivierten, zweiten, eine C=C-Doppelbindung aufweisenden und gegebenenfalls mindestens eine weitere Funktionalität aufweisenden Carbonsäure umgesetzt, und
(c) im Anschluss an die genannte Reaktion wird eine sulfonat- oder sulfatgruppenhaltige Verbindung oder ein Sulfit an die C=C-Doppelbindung des mit der zweiten funktionellen Gruppe umgesetzten Carbonsäurerestes addiert, derart, dass an dem mit der ersten der beiden funktionellen Gruppen umgesetzten (Meth)Acrylrest eine solche Addition nicht stattfindet, was sich auf verschiedenen Wegen, z.B. über das molare Verhältnis der miteinander umgesetzten Gruppen, sicherstellen lässt,
wobei es sich bei der zweiten eine C=C-Doppelbindung aufweisenden Carbonsäure um (Meth)Acrylsäure oder um eine andere doppelbindungshaltige Carbonsäure handeln kann.

Rein beispielhaft sei eine Reihe von möglichen Aminosilanen genannt, die in Schritt (a) als Ausgangsmaterial eingesetzt werden können:
(Aminoethylaminomethyl)phenylethyltrimethoxysilan, N-(2-Aminoethyl)-3-aminopropylmethyldimethoxysilan, N-(2-Aminoethyl-3-aminopropyl)trimethoxysilan, N-2-Aminoethyl-3-aminopropyltris(2-ethylhexoxy)silan, 6-(Aminohexylaminopropyl)-trimethoxysilan, N-(N'-(2 Aminoethyl)aminoethyl)-3-aminopropyl-trimethoxysilan, N-(N'-(2 Aminoethyl)aminoethyl)-3-aminopropyl-methyldimethoxysilan, N-(N'-(2 Aminoethyl)aminoethyl)-3-aminopropyl-triethoxysilan, N-(N'-(2 Aminoethyl)aminoethyl)-3-aminopropyl-methyldiethoxysilan, N-(N'-(2 Aminoethyl)aminoethyl)-3-aminopropyl-trimethylsilan, N-(N'-(2 Aminoethyl)aminoethyl)-3-aminopropyl-tris (methoxyethoxyethoxy)silan.

Stattdessen ließen sich vergleichbare Verbindungen mit entsprechenden Hydroxy- oder Thiolgruppen einsetzen. Solche sind beispielsweise in EP 0 779 890 A1 offenbart. Auch die Kohlenwasserstoffgruppe kann einen anderen Aufbau haben als in den oben gezeigten Beispielen.

### Variante (B):

(a) ein Silan mit einer über ein Kohlenstoffatom an das Si-Atom gebundenen Kohlenwasserstoffgruppe wird bereitgestellt, die mindestens einen reaktionsfähigen Heteroring trägt, ausgewählt unter den Dreiringen Oxacycyclopropyl- (=Epoxy-), Azacyclopropyl und Thiocyclopropyl und cyclischen Carbonaten (letztere können durch Umsetzung eines Epoxyrings mit CO₂, aber auch auf anderen Wegen erhalten werden, wie in DE 44 23811 ausführlich dargestellt),
(b) der Heteroring wird mit einem Sulfit oder einem Sulfat oder einer sulfonat- oder sulfatgruppenhaltigen Verbindung umgesetzt und
(c) zumindest die dabei freiwerdende OH-, SH- bzw. NH₂-Gruppe wird mit gegebenenfalls aktivierter (Meth)Acrylsäure umgesetzt.

Rein beispielhaft sei eine Reihe von möglichen Epoxidverbindungen genannt, die in Schritt (a) als Ausgangsmaterial eingesetzt werden können:
Glycidoxymethyltrimethoxysilan, Glycidoxymethyltriethoxysilan, 2-Glycidoxyethyltrimethoxysilan, 2-Glycidoxyethyltriethoxysilan, 3-Glycidoxypropyltrimethoxysilan, 3-Glycidoxypropyltriethoxysilan, 3-Glycidoxypropyltriacetoxysilan, 4-Glycidoxybutyltrimethoxysilan, 4-Glycidoxybutyltriethoxysilan, Glycidoxymethyl(methyl)dimethoxysilan, Glycidoxymethyl(ethyl)-dimethoxysilan, Glycidoxymethyl(phenyl)dimethoxysilan, Glycidoxymethyl(vinyl)dimethoxysilan, Glycidoxymethyl(dimethyl)methoxysilan, 2-Glycidoxyethyl(methyl)dimethoxysilan, 2-Glycidoxyethyl(ethyl)dimethoxysilan, 2-Glycidoxyethyl(dimethyl)methoxysilan, 3-Glycidoxypropyl(methyl)dimethoxysilan, 3-Glycidoxypropyl(ethyl)dimethoxysilan, 3-Glycidoxypropyl(dimethyl)methoxysilan, 4-Glycidoxybutyl(methyl)dimethoxysilan, 4-Glycidoxybutyl(ethyl)dimethoxysilan, 4-Glycidoxybutyl(dimethyl)methoxysilan, Bis-(glycidoxymethyl)dimethoxysilan, Bis-(glycidoxymethyl)diethoxysilan, Bis-(glycidoxyethyl)-dimethoxysilan, Bis-(glycidoxyethyl)diethoxysilan, Bis-(glycidoxypropyl)dimethoxysilan, Bis-(glycidoxypropyl)diethoxysilan, Tris-(glycidoxymethyl)methoxysilan, Tris-(glycidoxymethyl)ethoxysilan, Tris-(glycidoxyethyl)methoxysilan, Tris-(glycidoxyethyl)ethoxysilan, Tris-(glycidoxypropyl)methoxysilan, Tris-(glycidoxypropyl)ethoxysilan, Glycidylmethyltrimethoxysilan, Glycidylmethyltriethoxysilan, 2-Glycidylethyltrimethoxysilan, 2-Glycidylethyltriethoxysilan, 3-Glycidylpropyltrimethoxysilan, 3- Glycidylpropyltriethoxysilan, 3-Glycidylpropyltri-(methoxyethoxy)silan, 3-Glycidylpropyltriactoxysilan, 3,4-Epoxycyclohexylmethyltrimethoxysilan, 3,4-Epoxycyclohexylmethyltriethoxysilan, 3,4-Epoxycyclohexylethyltrimethoxysilan, 3,4-Epoxycyclohexylpropyltrimethoxysilan, 3,4-Epoxycyclohexylbutyltrimethoxysilan.

Stattdessen ließen sich vergleichbare Verbindungen mit entsprechenden Azacyclopropyl- und Thiocyclopropylgruppen einsetzen. Auch die Kohlenwasserstoffgruppe kann einen anderen Aufbau haben als in den oben gezeigten Beispielen.

### Variante (C):

(a) ein Silan mit einer über ein Kohlenstoffatom an das Si-Atom gebundenen Kohlenwasserstoffgruppe wird bereitgestellt, die eine Aminogruppe oder eine Mercaptogruppe aufweist,
(b) ein Alkenylsulfonat oder ein Sulfon wird mit der Aminogruppe bzw. der Mercaptogruppe zur Reaktion gebracht, und
(c) die in b. entstandene sekundäre Aminogruppe bzw. Thiogruppe wird mit gegebenenfalls aktivierter (Meth)acrylsäure umgesetzt.

Rein beispielhaft sei eine Reihe von möglichen Aminosilanen genannt, die in Schritt (a) als Ausgangsmaterial eingesetzt werden können:
4-Aminobutyldimethylmethoxysilan, 4-Aminobutyltriethoxysilan, Aminomethyltrimethylsilan, Aminophenyltrimethoxysilan, 3-(1-Aminopropoxy)-3,3-dimethyl-1-propenyltrimethoxysilan, 3-Aminopropyldiethylmethylsilan, 3-Aminopropyltris(methoxyethoxyethoxy)silan, 3-Aminopropyl-dimethylethoxysilan, 3-Aminopropylmethyldiethoxysilan, 3-Aminopropyltriethoxysilan, 3-Aminopropyltrimethoxysilan, 3-Aminopropyltris-(trimethylsiloxy)silan und ω-Aminoundecyltrimethoxysilan.

### Variante (D):

(a) ein (Meth)Acrylsilan mit mindestens zwei (Meth)Acrylgruppen, die über ein Kohlenstoffatom einer Kohlenwasserstoffgruppe an das Siliciumatom gebunden sind, wird bereit- bzw. nach dem Stand der Technik, z.B. gemäß DE 44 16 857, hergestellt, und
(b) eine sulfonat- oder sulfatgruppenhaltige Verbindung oder ein Sulfit wird im Unterschuss an die C=C-Doppelbindung eines oder mehrerer der (Meth)Acrylreste addiert, derart, dass an mindestens einem (Meth)Acrylrest im Molekül eine solche Addition nicht stattfindet, was sich auf verschiedenen Wegen, z.B. über das molare Verhältnis der miteinander umgesetzten Gruppen, sicherstellen lässt.

In den Varianten (A), (B) und (D) enthält die sulfonat- oder sulfatgruppenhaltige Verbindung vorzugsweise eine OH-, SH- oder NHR¹⁰-Gruppe mit R¹⁰ = Wasserstoff oder C₁-C₆-Alkyl. Beispiele für mercaptogruppenhaltige Sulfonate sind geradkettige oder cyclische Alkansulfonate oder Arylsulfonate, z.B. Mercaptoethansulfonate, Mercaptobutansulfonate, Mercaptocyclohexylsulfonate oder Mercaptobenzolsulfonate. Gängig sind deren Natriumsalze.

Eine dritte Ausgestaltung der Erfindung betrifft Verbindungen der Formel (II) mit f = 0 und g = 0, worin mindestens einer der Reste R³ oder R⁴ (letzterer im Falle von c = 1) einen funktionellen Substituenten trägt, der einen (Meth)Acrylrest und/oder eine saure Gruppe oder- in manchen Fällen - stattdessen oder zusätzlich eine Thioethergruppe aufweist. Dabei kann der funktionelle Substituent aus dem (Meth)Acrylrest oder der sauren Gruppe bestehen oder eine diesen Rest/diese Reste umfassende Einheit sein.

Um rein organische Verbindungen herzustellen, können die obigen Varianten (A), (B) und (D) in geeigneter Weise modifiziert werden, indem entsprechende Verbindungen ohne Silylrest als Ausgangsmaterialien eingesetzt werden. Hinsichtlich der Herstellungsvariante (A) ist anzumerken, dass man erfindungsgemäße Verbindungen erhält, wenn an das Ausgangsmaterial mindestens drei doppelbindungshaltige Säuren angebunden werden, was sich z.B. dadurch realisieren lässt, dass eine kohlenwasserstoffhaltige Ausgangsverbindung mindestens drei der in (A)a. genannten funktionellen Gruppen aufweist. Werden an diese drei Gruppen (Meth)Acrylreste angebunden, können entweder an eine davon oder an zwei davon eine sulfonat- oder sulfatgruppenhaltige Verbindung oder ein Sulfit addiert werden. Erfindungsgemäße Verbindungen erhält man auch, wenn man von einem Ausgangsmaterial mit nur zwei funktionellen Gruppen ausgeht, jedoch mindestens eine davon mit dem Anhydrid einer doppelbindungshaltigen Dicarbonsäure umsetzt. Bei Umsetzungen analog Variante (B) werden dann erfindungsgemäße Verbindungen erhalten, wenn der Heteroring mit einer sulfonat- oder sulfatgruppenhaltigen Verbindung derart umgesetzt wird, dass eine weitere Anknüpfungsstelle für die Anbindung eines zusätzlichen (Meth)Acrylats oder einer weiteren Säure ensteht, d.h. wenn z.B. ein Aminoalkansulfonat mit dem Dreiring umgesetzt wird. In Variante (D) wird anstelle eines (Meth)Acrylsilans eine organische Verbindung mit mindestens drei (Meth)Acrylgruppen eingesetzt, beispielsweise Trimethylolpropantriacrylat. Die schwefelhaltige Verbindung kann dann in einem molaren Verhältnis derart addiert werden, dass das Produkt entweder mindestens zwei (Meth)Acrylreste oder mindestens zwei Sulf(on)atreste enthält. Wenn in den Varianten (A), (B) oder (D) ein Thioalkansulf(on)at verwendet wird, entsteht eine Thioethergruppe, die in manchen Ausführungsformen als dritte funktionelle Gruppe neben dem mindestens einen (Meth)Acrylat und der mindestens einen Sulf(on)atgruppe angesehen werden kann, weil sie den Brechungsindex des daraus herstellbaren Kondensats erhöht, wie oben ausgeführt.

Sofern in den Herstellungsverfahren der Erfindung aktivierte Carbonsäuren eingesetzt werden sollen, kann die Aktivierung nach gängigen Methoden erfolgt; so können beispielsweise Säurechloride oder (intramolekulare oder intermolekulare) Anhydride eingesetzt werden.

Sofern in den Reaktionen der Erfindung anorganische Sulfonate oder Sulfate eingesetzt werden, eignen sich hierfür insbesondere die Alkali-, die Erdalkali- und die Ammoniumsalze. Unter den Alkalisalzen sind die des Natriums und des Kaliums und unter den Erdalkalisalzen sind die des Magnesiums und des Calciums bevorzugt. Die erfindungsgemäßen Silane können diese Salzgruppen oder die entsprechende freie Säurefunktion aufweisen.

Die verschiedenen Umsetzungen gemäß Variante (A) sollen nachstehend beispielhaft anhand der Reaktionen 1, 2 und 7 näher erläutert werden, wobei die Reaktionen 1 und 2 die Herstellung von Silanverbindungen und Reaktion 7 die Herstellung einer siliciumfreien Verbindung zeigen. Reaktion 7 folgt dabei dem Schema der Reaktion 2.

### Reaktion 1

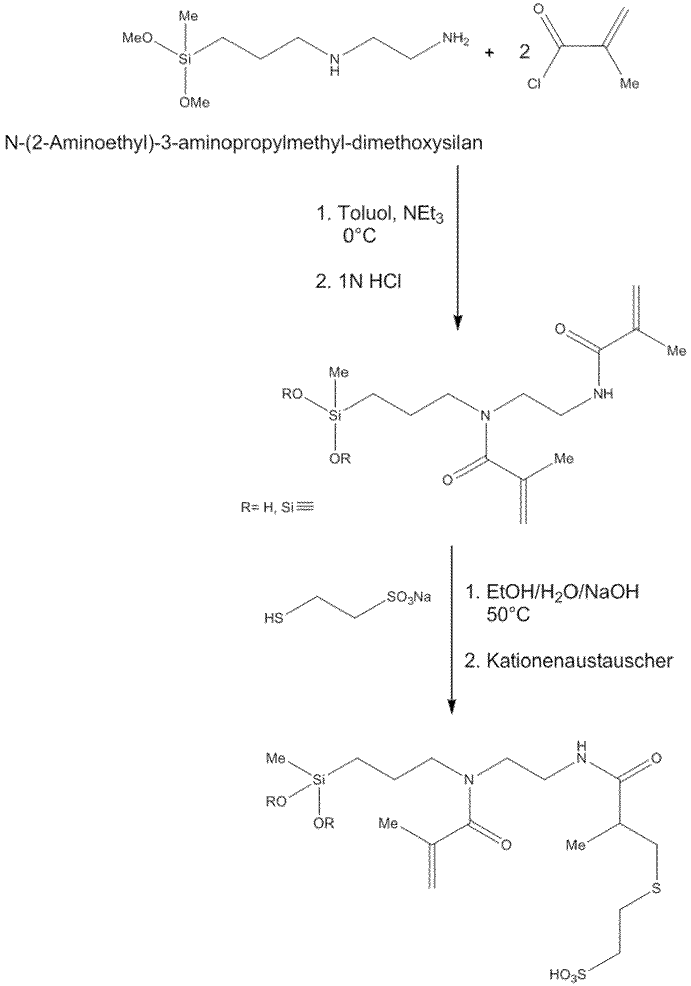

Für Reaktion 1 wurde ein Ausgangssilan mit einer Kohlenwasserstoffgruppe ausgewählt, die zwei Aminofunktionen trägt. Die Reaktion verläuft in vergleichbarer Weise, wenn anstelle des primären, endständigen Amins eine Hydroxy- oder eine Thiolgruppe vorhanden ist. Die sekundäre NH-Gruppe könnte fehlen; stattdessen könnte die an das Silicium gebundene Alkylengruppe ein durchgehendes Kohlenstoffskelett aufweisen, das an geeigneter Stelle (z.B. an der Position, an der sich im Beispiel die sekundäre NH-Gruppe befindet) mit NH₂, OH oder SH substituiert wäre. Das Ausgangssilan kann gegebenenfalls zusätzlich eine zweite sekundäre Aminogruppe aufweisen; in diesem Fall würden bei der Umsetzung mit Methacrylchlorid nicht nur zwei, sondern drei Acrylamidgruppen entstehen.

Die Länge und die Struktur der Kohlenwasserstoffgruppe im Molekül ist nicht kritisch und kann beliebig ausgewählt sein. So kann der Kohlenwasserstoff ein geradkettiges oder verzweigtes oder mindestens einen Cyclus aufweisendes Alkyl sein. Außerdem kann der Kohlenwasserstoff ggf. mit anderen Gruppen substituiert sein, die an den Umsetzungen nicht teilnehmen, oder beliebig durch Heteroatome oder Verknüpfungsgruppen unterbrochen sein.

Die in Reaktion 1 mit Me und OR bezeichneten Substituenten des Siliciums können je nach Bedarf beliebig ausgewählt sein, d.h., es kann eine für das gewünschte Kieselsäure(hetero)polykondensat geeignete Anzahl von hydrolytisch kondensierbaren Resten bzw. von als Netzwerkwandler fungierenden Resten (Alkyl-, Arylgruppen und dgl.) vorhanden sein. In speziellen Ausführungsformen können zwei die erfindungsgemäßen reaktiven Funktionen tragende Kohlenwasserstoffgruppen vorhanden sein; die Summe an hydrolytisch kondensierbaren Resten und als Netzwerkwandler fungierenden Resten beträgt dann 2.

Für die Herstellung des erfindungsgemäßen Silans wird einstufig oder zweistufig eine ausreichende Menge an (aktivierter) (Meth)Acrylsäure bereitgestellt, sodass ein Molekül mit zwei (Meth)Acrylsäureamid-Resten entsteht. Sollte das Ausgangssilan anstelle einer oder beider der Aminogruppen eine oder zwei Hydroxy- bzw. Thiolgruppen enthalten, würden sich jeweils die entsprechenden (Meth)Acrylsäureester- bzw. (Meth)Acrylsäurethioester bilden. Wenn das Ausgangssilan anstelle der sekundären Aminogruppe eine weitere primäre Aminogruppe enthält, entsteht eine Verbindung mit zwei primären (Meth)Acrylamiden.

Im zweiten Schritt der Reaktion wird eine der beiden durch die Anbindung der (Meth)Acrylsäuregruppen ins Molekül eingeführten Doppelbindungen für eine Thiol-en-Addition genutzt. Mit dieser Reaktion wird eine Sulfonsäuregruppe in das Molekül eingeführt. In vergleichbarer Weise lässt sich eine Sulfatgruppe einführen.

### Reaktion 2:

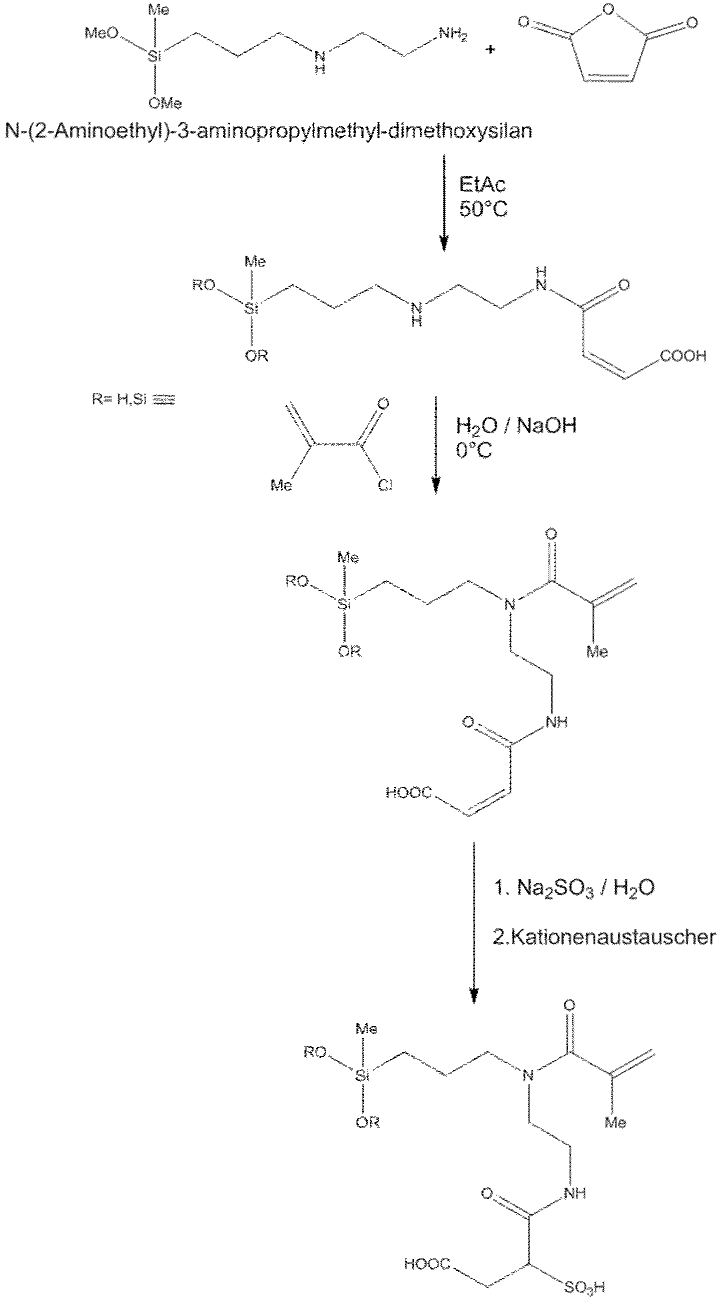

Hinsichtlich der einsetzbaren Ausgangsverbindungen für Reaktion 2 gilt das für Reaktion 1 Gesagte. Die Reaktionsführung unterscheidet sich von Reaktion 1 dadurch, dass in einem ersten Schritt nicht (Meth)Acrylsäure, sondern eine andere, ggf. aktivierte Carbonsäure eingesetzt wird, die eine C=C-Doppelbindung, vorzugsweise in Kombination mit einer C=O-Gruppe (d.h. ein Michael-System, enthaltend die Struktur C=C-C=O) aufweist. Im Beispiel wurde das Anhydrid der Maleinsäure verwendet. Aufgrund der etwas unterschiedlichen Reaktivität der primären und der sekundären Aminogruppen bindet diese, sofern sie nicht im Überschuss eingesetzt wird, in überraschender Weise ausschließlich an die primäre Aminogruppe; die dabei freiwerdende Carbonsäuregruppe ist nicht reaktiv genug, um an der sekundären Aminogruppe anzugreifen, so dass ausschließlich oder fast ausschließlich eine Produkt wie in Reaktion beispielhaft gezeigt entsteht. Dieses wird anschließend mit aktivierter (Meth)Acrylsäure umgesetzt. Mit Hilfe von Natriumsulfit lässt sich schließlich eine Sulfonatgruppe an die Doppelbindung des Maleinsäurerestes addieren, die bei Bedarf in bekannter Weise in eine Sulfonsäuregruppe überführt werden kann.

Das Produkt trägt zusätzlich eine freie Carbonsäuregruppe, die entweder für eine nochmals verbesserte Haft- und Ätzwirkung genutzt oder aber nach Bedarf weiter umgesetzt, z.B. komplexiert werden kann.

### Reaktion 7:

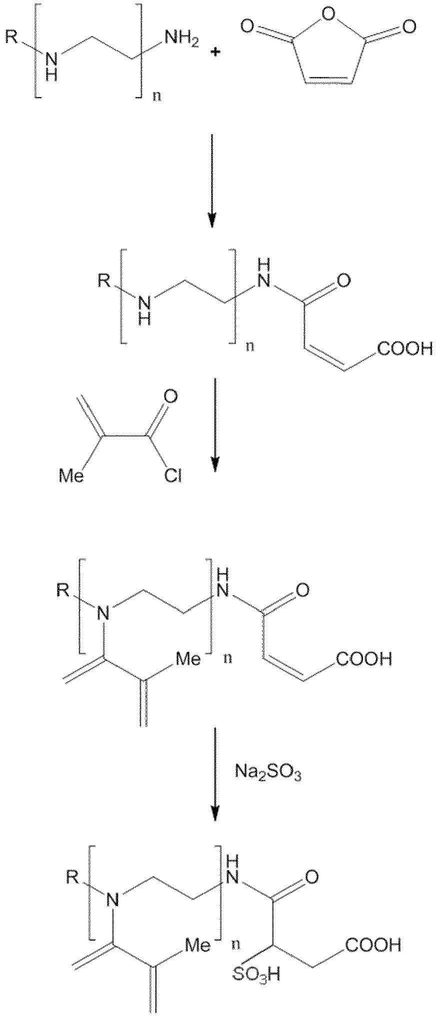

Diese Umsetzung zeichnet sich dadurch besonders aus, dass durch die Auswahl des Index n das Verhältnis von (Meth)Acrylgruppen zu Sulfonsäure- und Carbonsäuregruppen nach Bedarf eingestellt werden kann.

Eine Umsetzung gemäß Variante (B) soll nachstehend beispielhaft anhand der Reaktion 5 näher erläutert werden.

### Reaktion 5a und 5b:

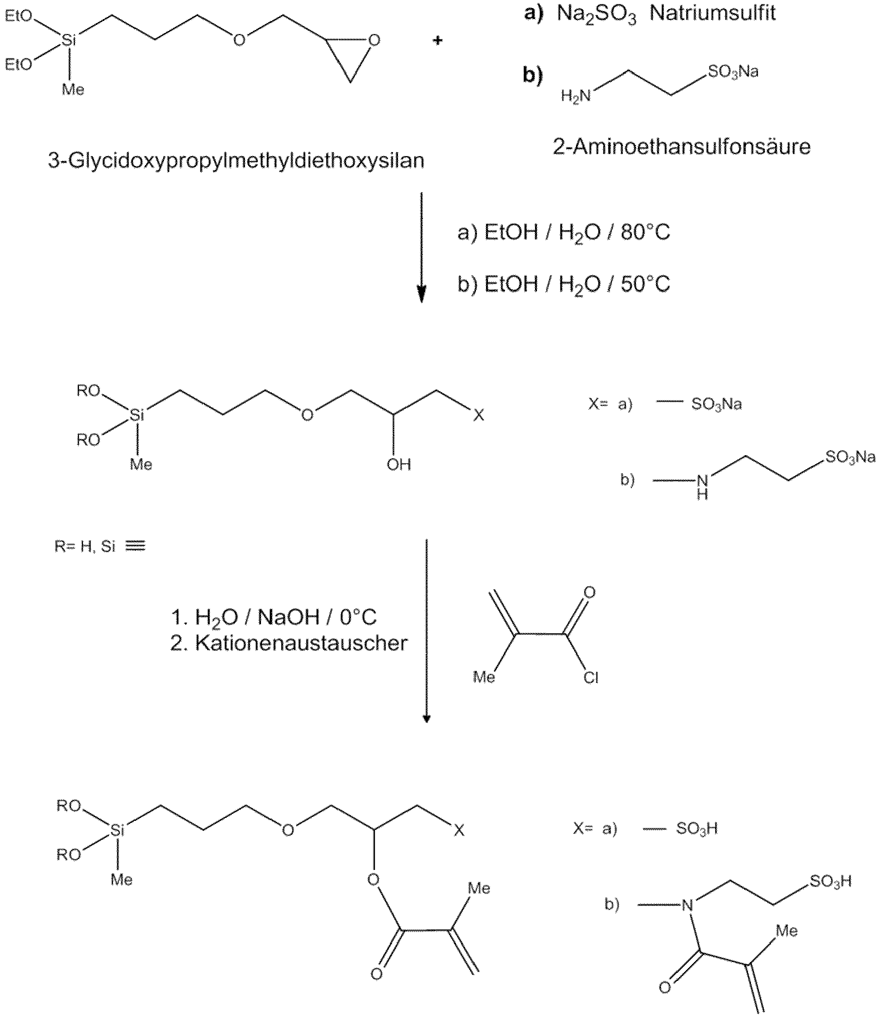

Hinsichtlich der Variabilität des Ausgangssilans sei auf die Ausführungen zu den Reaktionen 1 und 2 verwiesen, die in vergleichbarer Weise auch für die Reaktion 5 gelten. Zwingend ist an der Kohlenwasserstoffgruppe nur das Vorhandensein eines gespannten und damit reaktiven Heterorings. Im gewählten Beispiel ist das eine endständige Epoxygruppe. Die Reaktion ließe sich aber stattdessen auch mit einem Aziridin oder einer Thiocyclopropylgruppe durchführen. Dann würde im ersten Schritt anstelle einer freien Hydroxygruppe eine Amino- oder eine Thiolgruppe entstehen. Wird allerdings anstatt eines gespannten Dreirings ein cyclisches Carbonat eingesetzt, kann die Reaktion nur nach der in der Reaktion 5 gezeigten Variante b) erfolgen.

Wenn der Dreiring direkt mit einem Sulfit oder einem Sulfat umgesetzt wird, ergibt sich zwingend eine Ethylen- bzw. Ethylenoxybrücke zwischen der SO₃Na- oder SO₃H-Grupp und der Anknüpfungs stelle für die nachfolgende Veresterung der im ersten Schritt entstandenen Hydroxygruppe mit (aktivierter) (Meth)Acrylsäure. Reaktion 5 ist im Übrigen ein Beispiel dafür, dass auch zwei (Meth)Acrylatreste an eine Alkylsilangruppe angekoppelt werden können. Anstelle einer Aminoalkansulfonsäure kann in Reaktion 5 übrigens in allen Fällen auch eine Thio- oder eine Hydroxyalkansulfonsäure verwendet werden. Wird eine Thio- oder Hydroxyalkansulfonsäure eingesetzt, kommt es allerdings nicht zur Anbindung eines zweiten Methacrylatrestes; vielmehr bleibt der Rest, der in der Variante b) über die schlangenlinienförmige Verknüpfung angebunden ist, eine Thio- oder Oxyalkansulfonatgruppe. Auch dann, wenn (Meth)Acrylsäure im Unterschuss verwendet wird, kommt es zur Anbindung nur eines (Meth)Acrylatrestes; die Anbindung erfolgt an der basischsten der vorhandenen Gruppen, d.h. an der einzigen oder der basischsten Aminogruppe, sofern eine solche vorhanden ist.

Wird eine Thioalkansulfonsäure verwendet, entsteht eine Thioethergruppe. Ein Molekül gemäß dem Produkt von Reaktion 5 mit dieser Gruppe, aber ohne den Silylrest, kann unter manchen Aspekten ebenfalls als erfindungsgemäß angesehen werden.

Eine Umsetzung gemäß Variante (C) sollen nachstehend beispielhaft anhand der Reaktion 6 näher erläutert werden.

### Reaktion 6:

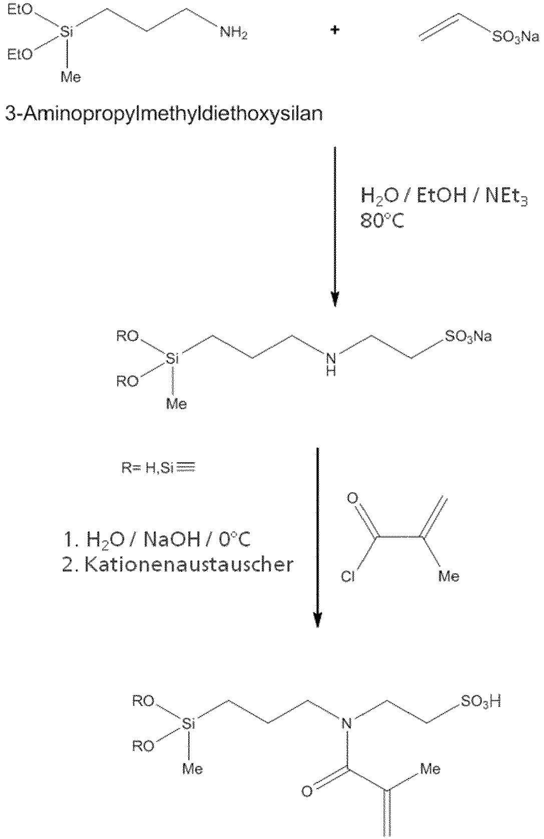

Hinsichtlich der Variabilität des Ausgangssilans sei wiederum auf die Ausführungen zu den Reaktionen 1 und 2 verwiesen, die in vergleichbarer Weise auch für die Reaktion 6 gelten. Die Länge und sonstige Gestalt der Alkylgruppe am Siliciumatom kann frei gewählt werden, sofern sie eine primäre Aminogruppe aufweist. An diese wird erst ein Alkenylsulfonat und anschließend (aktivierte) (Meth)Acrylsäure angekoppelt; das Sulfonat kann wie auch in den übrigen Reaktionsabfolgen anschließend in geeigneter Weise in die freie Sulfonsäuregruppe überführt werden.

Die voranstehenden Reaktionsbeispiele zeigen Umsetzungen zu Sulfonaten. Durch die Verwendung von Sulfaten wie in US 6,777,521 beschrieben anstelle von Sulfiten in den Umsetzungen gemäß Variante (B) lassen sich entsprechende Sulfatverbindungen erhalten. Eine Umsetzung gemäß Variante (D) soll nachstehend beispielhaft anhand von Reaktion 4 gezeigt werden:

### Reaktion 4:

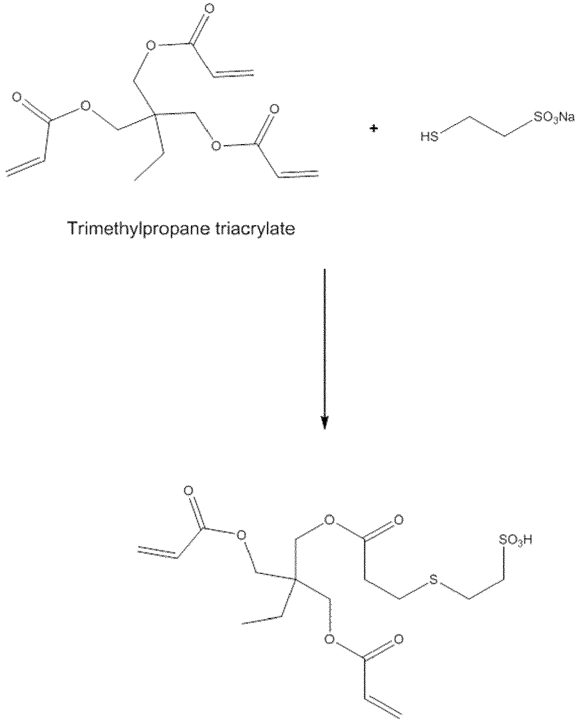

Diese Umsetzung zeichnet sich dadurch aus, dass es durch die Wahl der molaren Menge an Sulfonsäureverbindung möglich ist, Verbindungen mit zwei (Meth)Acrylresten und einem Sulfonsäurerest (bei Einsatz eines Mols Sulfonsäureverbindung pro Mol Methacrylat) oder, wenn die zweifache Molmenge an Sulfonsäureverbindung eingesetzt wird, Verbindungen mit einem (Meth)Acrylrest und zwei Sulfonatresten zu erhalten. Wird eine nichtstöchiometrische Menge an Sulfonat eingesetzt, lassen sich in beliebiger Weise Molekülmischungen erhalten.

Die voranstehenden Reaktionsbeispiele zeigen Umsetzungen zu Sulfonaten. Durch die Verwendung von Sulfaten wie in US 6,777,521 beschrieben anstelle von Sulfiten in den Umsetzungen gemäß Variante (B) lassen sich entsprechende Sulfatverbindungen erhalten.

Soweit es sich bei den erfindungsgemäßen Verbindungen um Silane handelt, können diese durch übliche Methoden (insbesondere das Sol-Gel-Verfahren) hydrolytisch kondensiert werden; es entstehen dabei Kieselsäurepolykondensate, auch als ORMOCER^{®}e bezeichnet. Auch diese werden von der Erfindung umfasst. Die Kondensationsreaktion kann in Gegenwart weiterer Silane der Formel SiR*ₐR**₄₋ₐ erfolgen, die aus dem Stand der Technik in sehr großer Zahl bekannt sind. R* bedeutet darin eine hydrolysierbare Gruppe, die das Einkondensieren des Silans in das Netzwerk ermöglicht, während R** ein beliebiger, nicht kondensierbarer Rest ist. Wenn bei der Kondensationsreaktion zusätzlich andere Metallverbindungen vorhanden sein sollen, z.B. Alkoxyverbindungen des Aluminiums, des Titans, des Zirkoniums oder des Zinns, entsteht ein Kieselsäure(hetero)polykondensat, bei dem die genannten Metallatome in das Si-O-Si-Netzwerk eingebunden sind. R* kann je nach Bedarf aber auch ein Halogenid wie Cl, Wasserstoff, Acyloxy mit vorzugsweise 2 bis 5 Kohlenstoffatomen, Alkylcarbonyl mit vorzugsweise 2 bis 6 Kohlenstoffatomen oder Alkoxycarbonyl mit vorzugsweise 2 bis 6 Kohlenstoffatomen sein. In manchen Fällen kann R* auch NR² mit R² gleich Wasserstoff, Alkyl mit vorzugsweise 1-4 Kohlenstoffatomen oder Aryl mit vorzugsweise 6-12 Kohlenstoffatomen sein.

Es kann wünschenswert sein, zusätzliche Metallverbindungen für die Einkondensierung in das anorganische Netzwerkt vorzusehen. Hierfür kommen insbesondere hydrolytisch kondensierbare Verbindungen von Metallen der III. und der IV. Hauptgruppe sowie der III. bis VI. Nebengruppe in Betracht, z.B. des Bors, des Aluminiums, des Titans des Germaniums, des Zirkoniums oder des Zinns. Diese Metallverbindungen sind in großer Zahl bekannt. In diesen Fällen entsteht ein Kieselsäure(hetero)polykondensat, bei dem die genannten Metallatome in das Si-O-Si-Netzwerk eingebunden sind. Die zusätzlichen Metallverbindungen sind häufig Alkoxyverbindungen; In spezifischen Ausführungsformen der Erfindung können die anderen Metallverbindungen jedoch ebenfalls ihrerseits reaktive Gruppen aufweisen. Besonders interessant für die vorliegende Erfindung sind dabei Komplexe, die ihrerseits (Meth)Acrylgruppen tragen, da diese bei einer nachfolgenden organischen Polymerisation in das organische Netzwerk mit eingebunden werden können.

Die Umsetzungen und damit die Einführung der Sulfonsäure- bzw. Sulfatgruppen können ganz oder teilweise statt auf der Silan- auf der Stufe nach der Polykondensation (meist einem Sol-Gel-Schritt), d. h. nach Aufbau der anorganischen Polymerstruktur, durchgeführt werden.

Unabhängig davon, ob die erfindungsgemäßen Verbindungen Silylgruppen enthalten oder nicht, können sie aufgrund der vorhandenen (Meth)Acrylgruppe(n) einer organischen Polymerisation unterworfen werden. Die hierdurch entstandenen, über die Polymerisation gehärteten Materialien werden ebenfalls von der Erfindung umfasst.

Selbstverständlich können auch kondensierte Silane weiterhin organisch gehärtet werden. Auch derart gehärtet Materialien, die ein anorganisches Si-O-Si-Netzwerk, ggf. mit zusätzlichen Kationen wie oben erwähnt, sowie ein Netzwerk enthalten, das aus der Polymerisation von Methacrylgruppen entstanden ist, werden von der Erfindung umfasst.

Die erfindungsgemäß einsetzbaren Synthesen zeichnen sich durch Einfachheit der Reaktionsführung, eine geringe Anzahl von Arbeitsschritten und gute Ausbeuten aus.

Wie teilweise bereits oben erwähnt, kann in spezifischen Ausführungsformen der Erfindung eine Verbindung mit mehr als einer Sulfonsäure- oder Schwefelsäuregruppe und/oder mit mehr als einer (Meth)Acrylgruppe substituiert sein. Durch das Vorhandensein von mehr als einer (Meth)Acrylgruppe kann das Netzwerk, das sich beim Polymerisieren bildet, noch engmaschiger werden. In diesem Zusammenhang ist zu erwähnen, dass über den Gehalt an polymerisierbaren Doppelbindungen der E-Modul des späteren organisch polymerisierten Polymer eingestellt werden kann, derart, dass das Polymer mehr oder weniger flexibel und dabei weniger hart oder härter ausfällt. Durch das Vorhandensein von mehr als einer Sulfonsäure- oder Schwefelsäuregruppe wird die Ätzwirkung des Materials weiter erhöht.

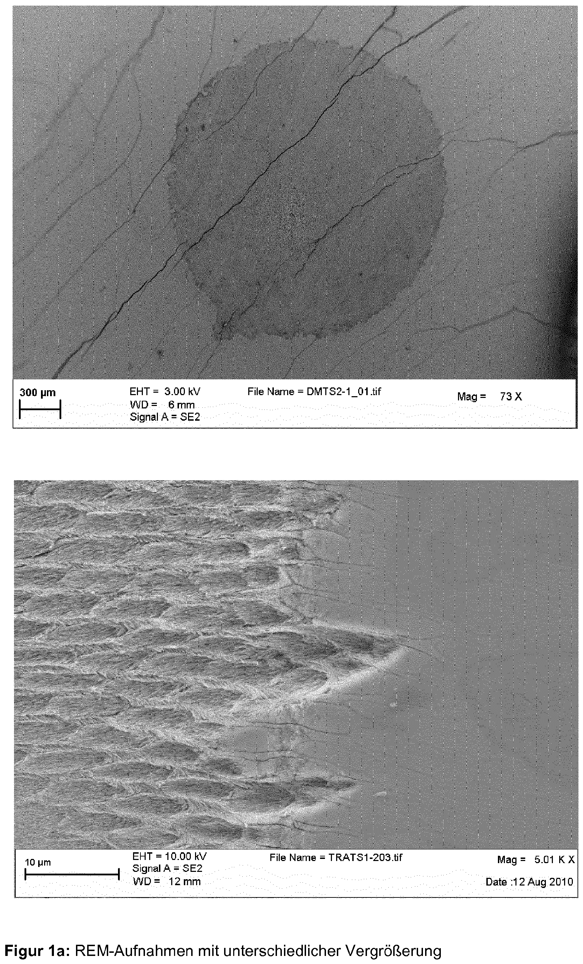

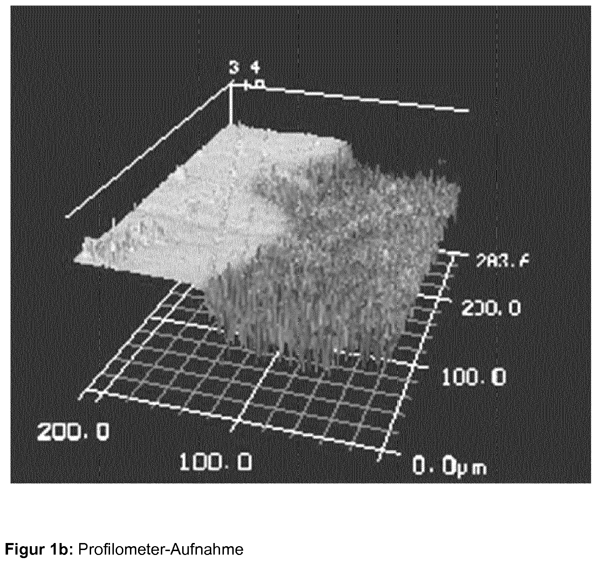

Die Erfinder konnten überraschend feststellen, dass bereits mit geringen Sulfonsäuregehalten eine enorme Ätzwirkung auf dem Zahngewebe beobachtet werden kann Dies lässt sich anhand eines Vergleichs der mittleren Rauheit der Schmelzoberfläche aufzeigen: Polierter Schmelz besitzt eine mittlere Rauheit von etwa 0,21µm, gemessen mit einem optischen Profilometer der Firma UBM. Mit einem phosphonsäurefunktionalisierten Kieselsäurepolykondensat aus Glycerin-1-methacryloyl-2-(siloxypropyl)carboxymethylphosphonsäure kann man Rauheiten im Bereich von 0,33µm erzielen. Mit Kondensaten der erfindungsgemäßen Verbindungen liegt die Rauheit im Bereich von über 0,45µm. Zahnschmelzaufnahmen sind in den **Figuren 1a** **und** **1b** gezeigt.

Die erfindungsgemäßen Verbindungen, und zwar sowohl die Silane als auch die rein organischen Verbindungen, sind in der Regel wasserlöslich, was in vielerlei Hinsicht vorteilhaft sein kann. Dies mag zu erwarten gewesen sein. Überrascht wurden die Erfinder jedoch davon, dass auch die aus den Silanen erzeugten Kieselsäurepolykondensate in der Regel wasserlöslich sind, obwohl sie ja eine Vielzahl von (Meth)Acrylatgruppen tragen. Dies hat für viele Anwendungen enorme Vorteile, wobei an vorderster Stelle medizinische Applikationen zu nennen sind. Denn die Kondensate können in wässrigem Medium angewendet, d.h. in beliebiger Form appliziert werden, ohne dass der Einsatz eines nichtwässrigen Lösungsmittels erforderlich wäre. Aber auch für industrielle Anwendungen sind wasserbasierte Reaktionen immer vorteilhaft, und zwar schon aus Gründen der Arbeitssicherheit und der Umweltverträglichkeit.

Die Möglichkeit, neben der Sulfonsäurefunktion weitere reaktive Gruppen in den erfindungsgemäßen Verbindungen auszubilden, erschließt zusätzliche Möglichkeiten. So haben Sulfonsäuregruppen eine stärkere Ätzwirkung als Carboxylgruppen, während diese komplexbildende Eigenschaften haben. Sofern zusätzliche Hydroxygruppen vorhanden sind, können diese entweder zur Verbesserung der Benetzung am Untergrund oder für weitere Umsetzungen genutzt werden, die die erfindungsgemäßen Verbindungen weiter modifizieren können. Ein Beispiel ist eine Komplexierung oder eine Umsetzung mit einer Dicarbonsäure (die z.B. mit Hilfe entsprechend aktivierter Säuremoleküle bewirkt werden kann).

Neben Polymerisationsgrad und Ätzwirkung besitzen die an den Verbindungen der Erfindung befindlichen Gruppen und Reste weitere Eigenschaften, die günstig für eine Reihe von Verwendungszwecken sind: Die Sulfonat- bzw. Sulfatgruppe ist ein Ladungsträger, weshalb Verwendungen als Elektrophoresegele, als Materialien für die Elektrotauchlackierung oder als die Leitfähigkeit oder die Antistatik modifizierende Materialien möglich sind. Des Weiteren kann die Gruppe als saurer Katalysator dienen, und zwar zum einen für den Sol-Gel Prozess im Falle einer hydrolytischen Kondensation erfindungsgemäßer Silane (ein späterer Abtrennungsschritt zur Katalysator-Abtrennung kann dann entfallen) und zum anderen im Hinblick auf den späteren Einsatz (ein Beispiel sind mesoporöse Membranen mit Sulfonsäuregruppen, die als Katalysator für chemische Prozesse in Brennstoffzellen dienen können). Die Gruppe sorgt ferner für eine gute Löslichkeit in polaren Medien. Insbesondere, aber nicht nur für dentale Zwecke dient sie als haftvermittelnde Gruppe für anorganische, organische sowie hybride Oberflächen. Genauso wie Carbonsäuregruppen kann sie aber auch ionische Bindungen ausbilden, wodurch sich z.B. Alkali-, Erdalkali-, Ammonium-, Ti-, Zr-, Sn-, Ca- und andere geeignete Kationen in Form ihrer Salze in das Polykondensat-Netzwerk einbinden lassen. Hierdurch lassen sich eine Reihe von Modifikation bzw. materialspezifischen Einstellungen erreichen, z.B. hinsichtlich der Röntgenopazität, der Brechzahl oder der Kontakttoxizität. Die Brechzahl wird darüber hinaus durch das Vorhandensein einer Thioethergruppe beeinflusst (erhöht). Durch die Sulfonat- bzw. Sulfatgruppen im Material erhält dieses im Übrigen eine antimikrobiellen Wirkung. Aber auch auf ganz anderen Gebieten lässt sich die Erfindung anwenden, weil man mit sulfonat- bzw. sulfatgruppenhaltigen Materialien z.B. protonenleitende Membranen, z.B. für fuel cells, bilden kann. Weiter kommen die Materialien in Betracht z.B. als Ionenaustauscher, als pseudostatische Phase in der elektrokinetischen Chromatographie oder als grenzflächenaktiver Stoff (Tensid).

Insbesondere durch die Kombination der Sulfonat- bzw. Sulfatgruppen und ggf. zusätzlich der -CO₂H-Gruppen mit polymerisierbaren/polyaddierbaren Doppelbindungen in einem Molekül bietet sich der Einsatz im Medizinsektor (spez. Dentalbereich) z. B. als Haftvermittler und als Matrixbestandteil für Zemente an. Die Einstellung der Brechzahl kann zur Erhöhung der Transluzenz des Dentalmaterials und damit zu dessen Angleichung an das natürliche Zahnmaterial beitragen.

Mit den erfindungsgemäßen funktionalisierten Verbindungen stehen Verbindungen zur Verfügung, die einerseits direkt verwendet werden können (z.B. für die Funktionalisierung von Oberflächen) und andererseits, sofern es sich um Silane handelt, als Ausgangsverbindungen für die Herstellung von anorganischen Hydrolysaten/Kondensaten (Harzsystemen) sowie von anorganisch/organischen Verbundpolymeren (Matrixsystemen) dienen, d. h. nach organischer Polymerisation/ Polyaddition (Härtung) mit unterschiedlichen Eigenschaften. Mit den silanfreien Monomeren lassen sich rein organische Polymermassen bzw. Polymere erhalten. Durch Einsatz von beliebigen Füllstoffen (Partikel, Fasern) wie z. B. den in DE 1064378, DE 19832965, DE 10018405, DE 1041038 sowie in DE102005018351 beschriebenen Partikeln werden entsprechende Komposite erhalten. Diese plastisch verarbeitbaren Komposite zeichnen sich durch mögliche sehr hohe Füllstoffgehalte (s. Nanohybridkomposite) in Kombination mit einer ausgezeichneten Verarbeitbarkeit aus. Somit können unterschiedliche Eigenschaften in weiten Bereichen sowohl bei den erfindungsgemäßen Verbindungen bzw. Silanen, den daraus erhältlichen Harzsystemen, Matrixsystemen oder rein organischen Polymeren sowie den gefüllten Systemen (Kompositen) eingestellt und den Erfordernissen angepasst werden.

Je nach dem vorgesehenen speziellen Verwendungszweck können dem Kieselsäure(hetero)polykondensat bzw. den organisch zu härtenden Verbindungen geeignete Additive wie Initiatoren, Färbemittel (Farbstoffe oder Pigmente), Oxidationsinhibitoren, Polymerisationsinhibitoren (für die Vermeidung einer vorzeitigen Polymerisation), Verlaufsmittel, UV-Absorber, Stabilisatoren, mikrobiozide Wirkstoffe oder dergleichen zugesetzt werden, wie es dem Fachmann bekannt ist. Beispiele für Polymerisationsinitiatoren sind Initiatoren für die radikalische Polymerisation, und zwar für die thermische Härtung wie Peroxide (z.B. Dibenzoylperoxid) oder Photoinitiatoren wie Benzophenon, Campherchinon oder Kombinationen von α-Diketonen mit Aminen als Reduktionsmittel, wie beispielsweise aus der DE 199 03 177 C2 bekannt. Für die duale Aushärtung von radikalisch und kationisch polymerisierbaren Systemen können insbesondere Diaryliodonium- oder Triarylsulfoniumsalze zugesetzt werden, für die die vorgenannte Druckschrift ebenfalls Beispiele angibt.

Ein gefülltes Dentalkomposit (d.h. ein organisch noch nicht vernetztes, gefülltes Harz aus hydrolysierten und kondensierten Silanen der Erfindung) kann, nachdem es für den vorgesehenen Zweck angewendet wurde, in geeigneter Weise organisch vernetzt und damit ausgehärtet werden. Dem dient vor allem eine organische Polymerisation der (Meth-)Acrylatgruppen. Auch die silanfreien Verbindungen können über eine Polymerisation dieser Gruppen in ein Polymer überführt werden. Dies ist eine radikalische Polymerisation, die üblicherweise unter Zusatz von Radikalstartern wie den oben erwähnten und ggf. bekannten Aktivatoren unter Belichtung mit z. B. sichtbarem Licht (Blaulicht; Dentalstrahler) d. h. photochemisch, thermisch oder redoxinduziert, aber auch im Rahmen von 2-Komponenten-Reaktionen oder anaerob erfolgt. Die Kombination von Selbsthärtung mit z. B. photoinduzierter bzw. thermischer Härtung ist ebenfalls möglich.

Der Einsatz solcher Materialien erstreckt sich u. a. auf die Verwendung in Form von Bulkmaterialien, Kompositen, Zementen, Klebstoffen, Vergussmassen, Beschichtungsmaterialien, Haftvermittlern, Bindemitteln für keramische Partikel (keramische Formgebungsverfahren), zur Herstellung bzw. Primung von Füllstoffen und Fasern, Einsatz im Reaktionsextruder und dergleichen für die verschiedensten Zwecke (insbesondere für medizinische-, aber auch für (mikro)optische- und (mikro)elektronische Anwendungen).

Nachstehend werden Herstellungsverfahren für die obigen Reaktionen beispielhaft angegeben.

### Reaktion 1:

Stufe 1: 5.11 g (0.024 mol) N-(2-Aminoethyl)-3-aminopropylmethyldimethoxysilan wurde in 5.21 g Triethylamin und 30 mL Toluol gelöst und auf 0°C temperiert. Anschließend wurden 5.0 mL (0.051 mol) Methacrylsäurechlorid in 30 ml Toluol hinzu getropft. Das Reaktionsgemisch wurde 3 h bei Raumtemperatur gerührt. Das Gemisch wurde zentrifugiert und die erhaltene Lösung zur Hydrolyse und Kondensation mit 1 N Salzsäure auf pH 1-2 eingestellt. Nach 24 h wurden die flüchtigen Bestandteile unter Vakuum entfernt.
Stufe 2: 3.92 g (0.013 mol) des Produkts aus Stufe 1 wurden in 30 mL Ethanol gelöst, die Lösung mit Natriumhydroxid auf pH 10 eingestellt und auf 60°C erwärmt. Anschließend wurden 1.93 g (0.015 mol) Natrium 2-Mercaptoethansulfonat gelöst in 40 mL H₂O zugetropft und für 4 h gerührt. Ethanol wurde unter Vakuum entfernt und die wässrige Lösung mit einem Kationenaustauscher behandelt. Die flüchtigen Bestandteile wurden unter Vakuum entfernt. Das Endprodukt ist in Wasser wieder löslich.

### Reaktion 2:

Stufe 1: 8.69 g (0.042 mol) N-(2-Aminoethyl)-3-aminopropylmethyldimethoxysilan wurden in 50 mL Ethylacetat gelöst und auf 50°C erwärmt. Eine Lösung aus 4.23 g (0.043 mol) Maleinsäureanhydrid in 30 mL Ethylacetat wurde zugetropft und 19 h gerührt. Das Gemisch wurde zentrifugiert und der Rückstand zweimal mit Ethylacetat gereinigt und unter Vakuum getrocknet.
Stufe 2: 6.06 g (0.021 mol) des Produkts der Stufe 1 wurden in 5 mL Wasser und 1.72 g NaOH gelöst und auf 0°C temperiert. 2.1 mL (0.021 mol) Methacrylsäure-chlorid wurden unter starkem Rühren langsam hinzu getropft und für 5 h bei 50°C gerührt. Die flüchtigen Bestandteile wurden anschließend unter Vakuum entfernt.
Stufe 3: 9.82 g (0.021 mol) des Produkts der Stufe 2 wurden in 20 mL Wasser gelöst und auf 60° erwärmt. Anschließend wurden 2.61 g (0.021 mol) Natriumsulfit unter Rühren zugetropft und für 24 h gerührt. Die wässrige Lösung wurde mit einem Kationenaustauscher behandelt und die flüchtigen Bestandteile unter Vakuum entfernt. Das Produkt lässt sich in Wasser wieder lösen.

### Reaktion 5:

a)
   Stufe 1: In 30 mL H₂O wurden 5.04 g (0.040 mol) Natriumsulfit gelöst und auf 80°C erhitzt. Eine Lösung aus 9.96 g (0.040 mol) 3-Glycidoxypropylmethyldiethoxysilan in 10 mL Ethanol wurde hinzu getropft und für 3 h unter Rückfluss gerührt. Nach Abdampfen von Ethanol wurde die wässrige Phase mit Ethylacetat gereinigt und die flüchtigen Bestandteile anschließend unter Vakuum entfernt.
   Stufe 2: 5.04 g (0.016 mol) des Produkts der Stufe 1 wurde in 10 mL Wasser und 2.79 g (0.070 mol) NaOH gelöst und auf 0°C temperiert. 4.0 mL (0.016 mol) Methacrylsäurechlorid wurden anschließend zugetropft und das Reaktionsgemisch für 4 h bei 30°C gerührt. Die Lösung wurde mit Ethylacetat gereinigt, die wässrige Phase mit einem Kationenaustauscher behandelt und die flüchtigen Bestandteile anschließend unter Vakuum entfernt. Das Produkt lässt sich in Wasser wieder lösen.
b)
   Stufe 1: 3.38 g (0.027 mol) 2-Aminoethansulfonsäure wurden in 40 mL H₂O gelöst und mit 1 N NaOH-Lösung auf pH 14 eingestellt. Eine Lösung aus 6.81 g (0.027 mol) 3-Glycidoxypropylmethyldiethoxysilan in 30 mL Ethanol wurden bei 50°C zugetropft und für 3 h gerührt. Danach wurde Ethanol unter Vakuum entfernt und die wässrige Lösung mit Ethylacetat gereinigt. Die flüchtigen Komponenten wurden anschließend unter Vakuum entfernt.
   Stufe 2: 5.12 g (0.014 mol) des Produkts der Stufe 1 wurde in 10 mL Wasser und 2.58 g (0.065 mol) NaOH gelöst und auf 0°C temperiert. 1.5 mL (0.016 mol) Methacrylsäurechlorid wurden zugetropft und das Reaktionsgemisch für 4 h bei 30°C gerührt. Die wässrige Lösung wurde mit Ethylacetat gereinigt und mit einem Kationenaustauscher behandelt. Die flüchtigen Komponenten wurden anschließend unter Vakuum entfernt. Das Produkt lässt sich in Wasser wieder lösen.

### Reaktion 6:

Stufe 1: 5.14 g (0.027 mol) 3-Aminopropylmethyldiethoxysilan wurden in 30 mL Ethanol und 1.65 g (0.016 g) Triethylamin gelöst und auf 70°C erhitzt. Anschließend wurden 14 mL (0.027 mol) einer 25% wässrigen Natriumvinylsulfonat-Lösung zugetropft und für 24 h gerührt. Ethanol wurde anschließend unter Vakuum entfernt und die wässrige Lösung mit Ethylacetat gewaschen. Die flüchtigen Komponenten wurden anschließend unter Vakuum entfernt.
Stufe 2: 5.73 g (0.018 mol) des Produkts der Stufe 1 wurde in wurde in 10 mL Wasser und 2.92 g (0.075 mol) NaOH gelöst und auf 0°C temperiert. 1.8 mL (0.018 mol) Methacrylsäurechlorid wurden zugetropft und das Reaktionsgemisch für 5 h bei 30° gerührt. Das Lösungsmittel wurde anschließend unter Vakuum entfernt. Die wässrige Lösung wurde mit Ethylacetat gereinigt und mit einem Kationenaustauscher behandelt. Die flüchtigen Komponenten wurden anschließend unter Vakuum entfernt. Das Produkt lässt sich in Wasser wieder lösen.

### Reaktion 4:

1.50 g (0.005 mol) Trimethylpropantriacrylat und 13.9 mg (0.06 Gew.%) Butylhydroxytoluol wurden in 20 mL Ethanol gelöst und auf 40°C erwärmt. 0.87 g (0.005 mol) Natrium 2-Mercaptoethansulfonat wurden in 20 mL Wasser gelöst und zugetropft. Das Reaktionsgemisch wurde 4 h gerührt und Ethanol anschließend bei 40°C unter Vakuum entfernt. Die wässrige Lösung wurde danach mit Ethylacetat gereinigt, mit einem Kationenaustauscher behandelt und die flüchtigen Bestandteile unter Vakuum entfernt. Das Produkt kann wieder in Wasser gelöst werden.

## Patentansprüche

1. Verbindung, umfassend mindestens drei Funktionalitäten, nämlich (a) eine Sulfonat- oder Sulfatgruppe der Formel -(O)_{d}-SO₃M mit d = 0 oder 1 und M = Wasserstoff oder ein einwertiges Metallkation oder der entsprechende Anteil eines mehrwertigen Metallkations, (b) einen (Meth)Acrylrest, sowie (c) entweder (c1) eine Carbonsäurefunktion und/oder (c2) eine Thioethergruppe, mit der Maßgabe, dass in einer siliciumfreien Verbindung eine Sulfonat- oder Sulfatgruppe und ein (Meth)Acrylatrest über einen kohlenwasserstoffhaltigen Rest voneinander getrennt sind, dessen Kohlenstoffkette entweder durch O, S oder NH unterbrochen ist oder eine Verknüpfungsgruppe enthält.

2. Verbindung nach Anspruch 1, bei der es sich um ein Silan der Formel (I)
R⁷ₐR²_{b}SiZ_{4-a-b} (I)
handelt, worin R⁷ ein hydrolytisch kondensierbarer Rest ist, R² substituiertes oder unsubstituiertes, geradkettiges, verzweigtes oder einen Cyclus aufweisendes Alkyl, Aryl, Arylalkyl, Alkylaryl oder Alkylarylalkyl ist oder ein entsprechendes Alkenyl ist, dessen Kohlenstoffkette in allen Fällen gegebenenfalls durch -O-, -S-, -NH-, -S(O)-, -C(O)NH-, - NHC(O)-, -C(O)O-, -C(O)S, -NHC(O)NH- oder C(O)NHC(O)-Gruppen unterbrochen sein kann, die ggf. in beide möglichen Richtungen weisen können, Z ein Rest ist, in welchem mindestens eine (Meth)Acrylgruppe und mindestens entweder eine Sulfonat- oder eine Sulfatgruppe unmittelbar oder mittelbar über eine unsubstituierte oder substituierte Kohlenwasserstoffgruppe mit einer Si-C-Bindung am Siliciumatom gebunden vorliegen, a 1, 2 oder 3 ist, b 0, 1 oder 2 ist und a+b zusammen 2 oder 3 sind.

3. Silan nach Anspruch 1 oder 2 mit der Formel 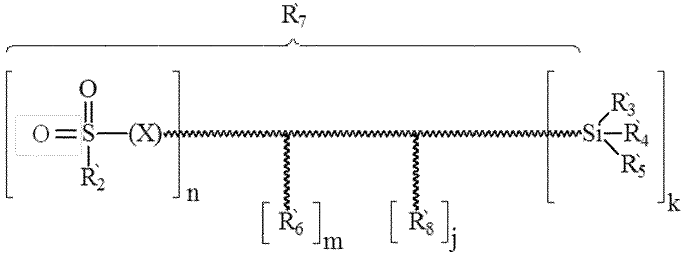 worin die Gruppen und indices die folgende Bedeutung haben:
R'₂ steht für OH oder ein Salz -OM mit M = einwertiges Metallkation oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium, X bedeutet Sauerstoff oder ist nicht vorhanden,
R'₃, R'₄, R'₅ sind entweder Reste, die unter Ausbildung von Si-O-Si-Brücken einer hydrolytischen Kondensation unterworfen werden können oder Reste, die über ein Kohlenstoffatom an das Siliciumatom gebunden sind;
R'₆ ist ein doppelbindungshaltiger Rest,
R'₇ bezeichnet den organischen Rest, der sowohl die Sulf(on)atgruppe als auch den doppelbindungshaltigen Rest aufweist; dieser ist über ein Kohlenstoffatom an das Silicium gebunden,.
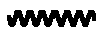 bezeichnet einen organischen Rest, der durch mindestens eine Verknüpfungsgruppe, ausgewählt unter -C(O)O-, -C(O)NH-, -NHC(O)-, -NHC(O)O, -C(O)NHC(O), -NHC(O)NH- -S(O)- oder die entsprechenden schwefelhaltigen Gruppen oder ein Sauerstoffatom, ein Schwefelatom oder eine sekundäre oder tertiäre Aminogruppe unterbrochen sein kann,
R'₈ bedeutet -CO₂H oder OH.
n, m und j bedeuten jeweils mindestens 1, können aber in bestimmten Fällen 2 oder 3 sein oder sogar einen noch höheren Wert annehmen.

4. Verbindung nach Anspruch 1 mit der Formel (II) 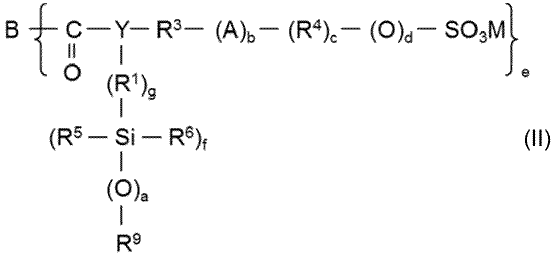 worin
R¹ ein zweibindiger, gegebenenfalls durch ein oder mehrere Sauerstoffatome, Schwefelatome, Estergruppen, Aminogruppen oder Amidgruppen unterbrochener Kohlenwasserstoffrest ist, der, sofern f = 1 ist, über ein Kohlenstoffatom am Siliciumatom gebunden ist,
R⁹ H oder Alkyl ist und zusätzlich im Falle von a = 0, f =1 und g =1
ein hydrolytisch kondensierbarer Rest oder 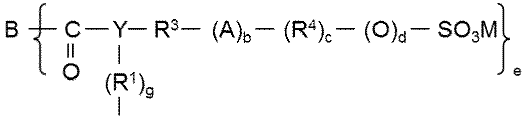 sein kann,
R³ ein unsubstituiertes oder mit einer funktionellen Gruppe substituiertes, geradkettiges, verzweigtes oder mindestens einen Cyclus aufweisendes Alkylen ist,
A eine Verknüpfungsgruppe darstellt,
R⁴ eine gegebenenfalls durch O, S, NH oder NR⁸ unterbrochene und/oder gegebenenfalls funktionell substituierte Kohlenwasserstoffgruppe, vorzugsweise ein solches Alkylen bedeutet,
M Wasserstoff oder ein einwertiges Metallkation oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium ist,
R⁵ und R⁶ unabhängig voneinander entweder unter Hydrolysebedingungen kondensierbare Reste oder substituiertes oder unsubstituiertes, geradkettiges, verzweigtes oder mindestens einen Cyclus aufweisendes Alkyl, Aryl, Arylalkyl, Alkylaryl oder Alkylarylalkyl sind, ausnahmsweise stattdessen aber auch ein entsprechendes Alkenyl, Arylalkenyl oder Alkenylaryl sein können,
R⁸ Alkyl oder Alkenyl mit vorzugsweise 1 bis 6 bzw. 2 bis 6 Kohlenstoffatomen oder ein (Meth)Acrylrest ist,
B Vinyl, 2-Allyl oder, im Falle von e > 1, ein organischer Rest mit e Vinylgruppen ist, die jeweils an eine in der geschweiften Klammer befindliche Gruppe gebunden vorliegen,
Y ein Stickstoffatom, -O-CH=, -S-CH= oder -NH-CH= ist, wobei jeweils das Sauerstoffatom, das Schwefelatom bzw. die NH-Gruppe eine Bindung zur benachbarten C(O)-Gruppe aufweist,
a = 0 oder 1 ist
b = 0 oder 1 ist
c = 0 oder 1 ist
d = 0 oder 1 ist, und
e = 1, 2 oder 3 ist
f = 0 oder 1 ist und
g = 0 oder 1 ist,
wobei dann, wenn f = 1 ist, a und g beide ≠ 0 sind und
wobei dann, wenn f = 0 ist, zumindest einer der Reste R³ oder R⁴ einen funktionellen Substituenten trägt, der einen (Meth)Acrylrest oder eine saure Gruppe aufweist oder R⁴ ein mindestens durch S unterbrochenes Alkylen bedeutet.

5. Verbindung nach einem der voranstehenden Ansprüche, worin mindestens einer der Reste R³ und R⁴ substituiert ist mit einem Rest R¹⁰COOM und gegebenenfalls mit mindestens einer Hydroxygruppe und/oder mit einem Rest SO₃M, worin M wie in Anspruch 1 definiert ist, worin R¹⁰ eine chemische Bindung oder ein C₁-C₆-Alkylenrest ist.

6. Verbindung nach Anspruch 4, worin f = 0 ist und g = 0 ist und a = 0 ist.

7. Verfahren zum Herstellen einer Verbindung mit der Formel (II) wie in Anspruch 4 definiert, **dadurch gekennzeichnet, dass**
(a) eine Kohlenwasserstoffverbindung bereitgestellt wird, die mindestens zwei funktionelle Gruppen trägt, ausgewählt unter primären Aminen, sekundären Aminen, Hydroxygruppen und Thiolgruppen,
(b) eine erste der beiden funktionellen Gruppen mit gegebenenfalls aktivierter (Meth)Acrylsäure und die zweite der beiden funktionellen Gruppen mit einer gegebenenfalls aktivierten, zweiten, eine C=C-Doppelbindung aufweisenden Carbonsäure umgesetzt werden und
(c) im Anschluss an die genannte Reaktion eine sulfonat- oder sulfatgruppenhaltige Verbindung oder ein Sulfit an die C=C-Doppelbindung des mit der zweiten funktionellen Gruppe umgesetzten Carbonsäurerestes addiert wird, derart, dass an dem mit der ersten der beiden funktionellen Gruppen umgesetzten (Meth)Acrylrest eine solche Addition nicht stattfindet,
wobei es sich bei der zweiten eine C=C-Doppelbindung aufweisenden Carbonsäure um (Meth)Acrylsäure oder um eine andere doppelbindungshaltige Carbonsäure handeln kann.

8. Verfahren zum Herstellen einer Verbindung der Formel (II) wie in Anspruch 4 definiert, **dadurch gekennzeichnet, dass**
(a) eine Kohlenwasserstoffverbindung bereitgestellt wird, die mindestens einen Heteroring trägt, ausgewählt unter Oxacycyclopropyl, Azacyclopropyl, Thiocyclopropyl und einem cyclischen Carbonat,
(b) der Heteroring mit einem Sulfit oder einer sulfonat- oder sulfatgruppenhaltigen Verbindung umgesetzt wird und
(c) zumindest die dabei freiwerdende OH-, SH- bzw. NH₂-Gruppe mit gegebenenfalls aktivierter (Meth)Acrylsäure umgesetzt wird.

9. Verfahren nach einem der Anspruch 7 oder 8, **dadurch gekennzeichnet, dass** die sulfonat- oder sulfatgruppenhaltige Verbindung eine OH-, SH- oder NHR¹¹-Gruppe mit R¹¹ = Wasserstoff oder C₁-C₆-Alkyl, insbesondere eine SH-Gruppe aufweist.

10. Verfahren zum Herstellen einer Verbindung der Formel (II) wie in Anspruch 4 definiert, **dadurch gekennzeichnet, dass**
(a) eine organische Verbindung mit mindestens drei (Meth)Acrylgruppen bereitgestellt wird, und
(b) eine sulfonat- oder sulfatgruppenhaltige Verbindung, die eine SH-Gruppe aufweist, im Unterschuss an die C=C-Doppelbindung eines oder mehrerer der (Meth)Acrylreste addiert wird, derart, dass an mindestens einem (Meth)Acrylrest im Molekül eine solche Addition nicht stattfindet.

11. Verfahren zum Herstellen einer Verbindung der Formel (II) wie in Anspruch 4 definiert, **dadurch gekennzeichnet, dass**
(a) ein (Meth)Acrylsilan mit mindestens zwei (Meth)Acrylgruppen, die über ein Kohlenstoffatom einer Kohlenwasserstoffgruppe an das Siliciumatom gebunden sind, bereitgestellt wird, und
(b) eine sulfonat- oder sulfatgruppenhaltige Verbindung, die eine SH-Gruppe aufweist, im Unterschuss an die C=C-Doppelbindung eines oder mehrerer der (Meth)Acrylreste addiert wird, derart, dass an mindestens einem (Meth)Acrylrest eine solche Addition nicht stattfindet.

12. Verfahren zum Herstellen einer Verbindung wie in Anspruch 6 definiert, **dadurch gekennzeichnet, dass** eine organische Verbindung, die mindestens eine (Meth)Acrylrest und mindestens einen Alkenylcarboxylrest aufweist, mit einer sulfonat- oder sulfatgruppenhaltigen Verbindung oder einem Sulfit umgesetzt wird, dass diese(s) an die C=C-Doppelbindung des Alkenylcarboxylrestes addiert wird, derart, dass an dem (Meth)Acrylrest eine solche Addition nicht stattfindet, wobei es sich bei dem mindestens einen Alkenylcarboxylrest um einen zweiten (Meth)Acrylrest oder um den Rest einer anderen Alkenylcarbonsäure handeln kann.

13. Kieselsäure(hetero)polykondensat, **dadurch gekennzeichnet, dass** es durch hydrolytische Kondensation unter Verwendung eines Silans der Formel (I), (la) oder (II) entstanden ist.

14. Kieselsäure(hetero)polykondensat nach Anspruch 13, **dadurch gekennzeichnet, dass** es unter der zusätzlichen Verwendung mindestens einer hydrolytisch kondensierbaren Metallverbindung eines Metalls entstanden ist, ausgewählt unter Metallen der III. und der IV. Hauptgruppe sowie der III. bis VI. Nebengruppe.

15. Kieselsäure(hetero)polykondensat nach einem der Ansprüche 13 oder 14, **dadurch gekennzeichnet, dass** es wasserlöslich ist.

16. Polymerisat, erhalten aus oder unter Verwendung mindestens einer Verbindung wie in einem der Ansprüche 1 bis 6 definiert durch Polymerisation mindestens eines Teils von deren (Meth)Acrylgruppen, wobei das Polymerisat optional zusätzlich ein anorganisch kondensiertes Netzwerk aus oder mit Si-O-Si-Brücken aufweist.

17. Verwendung eines Polymerisats nach Anspruch 16 oder eines Kieselsäure(hetero)polykondensats nach einem der Ansprüche 13 bis 15 für dentale Zwecke, insbesondere als Dentaladhäsiv.

## Claims

1. A compound comprising at least three functionalities, namely (a) a sulfonate group or a sulfate group of the formula -(O)_{d}-SO₃M with d = 0 or 1 and with M = hydrogen or a monovalent metal cation or the corresponding portion of a multivalent metal cation; (b) a (meth)acryl residue; and (c) either (c1) a carboxylic acid function and/or (c2) a thioether group; with the proviso that, when the compound is free of silicon, a sulfonate group or sulfate group and a (meth)acryl residue are separated from each other by a hydrocarbon-containing residue having a carbon chain which either is interrupted by O, S, or NH or contains a linking group.

2. The compound according to claim 1 that is a silane of the formula (I)
R⁷ₐR²_{b}SiZ_{4-a-b} (I)
wherein R⁷ is a hydrolytically condensable residue; R² is an alkyl, aryl, arylalkyl, alkylaryl or alkylarylalkyl that is substituted or unsubstituted, straight-chain, branched or comprises a cyclic structure, or is a corresponding alkenyl whose carbon chain in all cases optionally can be interrupted by -O-, -S-, -NH-, -S(O)-, -C(O)NH-, -NHC(O)-, - C(O)O-, -C(O)S, -NHC(O)NH-, or C(O)NHC(O) groups which can optionally be oriented in both possible directions; Z is a residue in which are present at least one (meth)acryl group and either at least a sulfonate group or a sulfate group that are bonded directly or indirectly by an unsubstituted or substituted hydrocarbon group via an Si-C bond to the silicon atom; a is 1, 2 or 3; b is 0, 1 or 2; and the sum of a+b is 2 or 3.

3. A silane according to claim 1 or 2 represented by the formula 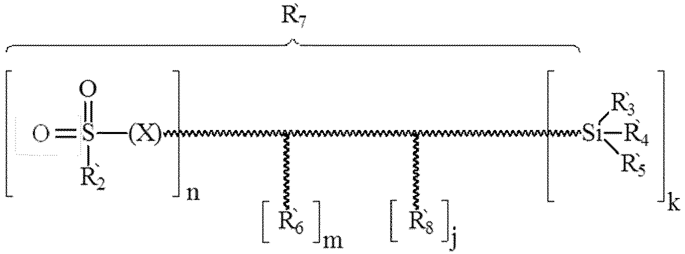 wherein the groups and indices have the following meaning:
R'₂ represent OH or a salt-OM wherein M is a monovalent metal cation or the corresponding part of a multivalent cation, preferably selected from among alkali cations and alkaline earth metal cations, in particular from among Na, K, ½ Ca, ½ Mg or ammonium,
X represent oxygen or is not present,
R'₃, R'₄ R'₅ are either residues which can be subjected to a hydrolytic condensation to form Si-O-Si linkages, or residues which are linked to the silicon atom via a carbon atom;
R'₆ is a double bond-containing residue,
R'₇ denotes the organic residue comprising the sulf(on)ate group and the double bond-containing residue; and is linked to the silicon atom via a carbon atom,
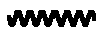 denotes an organic residue which can be interrupted by at least one linking group selected from -C(O)O- groups, -C(O)NH- groups, -NHC(O)- groups, -NHC(O)O groups, -C(O)NHC(O) groups, -NHC(O)NH- groups, -S(O)- groups or the corresponding sulfur-containing groups, or an oxygen atom, a sulfur atom or a secondary or tertiary amino group,
R'₈ represents -CO₂H or OH,
n, m and j each represent at least 1 but can in certain cases have a value of 2 or 3 or even a higher value.

4. The compound according to claim 1 represented by the formula (II) 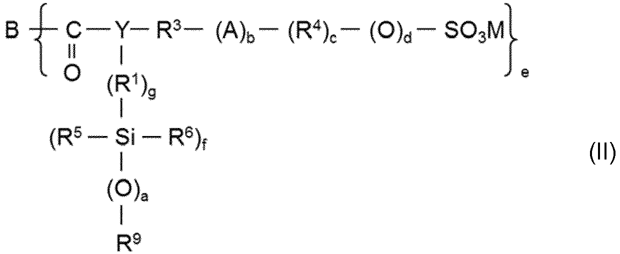 wherein
R¹ is a bivalent hydrocarbon residue which is optionally substituted by one or more oxygen atoms, sulfur atoms, ester groups, amino groups or amide groups, and which, provided that f = 1, is bonded by a carbon atom to the silicon atom,
R⁹ is H or alkyl and, in case of a = 0, f =1, and g =1, can be additionally a hydrolytically condensable residue or 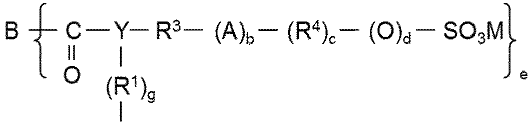 wherein R³ is an alkylene that is straight-chain, branched or has at least one cyclic structure, and is unsubstituted or substituted with a functional group,
A is a linking group,
R⁴ is a hydrocarbon group that is optionally interrupted by O, S, NH or NR⁸ and/or optionally functionally substituted, preferably is an alkylene of this type,
M is hydrogen or a monovalent metal cation or the corresponding portion of a multivalent metal cation, preferably selected from alkali cations and alkaline earth cations, in particular Na, K, ½ Ca, ½ Mg, or ammonium,
R⁵ and R⁶, independently of each other, are either residues that are condensable under hydrolysis conditions or alkyl, aryl, arylalkyl, alkylaryl or alkylarylalkyl substituted or unsubstituted, straight-chain, branched or having at least one cyclic structure, in exceptions instead also a corresponding alkenyl, arylalkenyl, or alkenylaryl,
R⁸ is alkyl with preferably 1 to 6 or alkenyl with preferably 2 to 6 carbon atoms or a (meth)acryl residue,
B is vinyl, 2-allyl or, in case of e > 1, an organic residue with e vinyl groups that are present in each case bonded to a group located in the curly brackets,
Y is a nitrogen atom, -O-CH=, -S-CH= or -NH-CH=, wherein in each case the oxygen atom, the sulfur atom or the NH group has a bond to the adjacent C(O) group,
a is = 0 or 1,
b is = 0 or 1,
c is = 0 or 1,
d is = 0 or 1, and
e is = 1, 2 or 3
f is = 0 or 1 and
g is = 0 or 1,
wherein, when f is = 1, then a and g both are ≠ 0, and
wherein, when f is = 0, then at least one of the residues R³ or R⁴ carries a functional substituent which has a (meth)acryl residue or an acidic group or R⁴ is an alkylene that is interrupted at least by S.

5. The compound according to one of the preceding claims, wherein at least one of the residues R³ and R⁴ is substituted with a residue R¹⁰COOM and optionally with at least one hydroxyl group and/or with a residue SO₃M, wherein M is defined as in claim 1, wherein R¹⁰ is a chemical bond or a C₁-C₆ alkylene residue.

6. The compound according to claim 4, wherein f = 0 and g = 0 and a = 0.

7. A method for preparing a compound with the formula (II) as defined in claim 4, **characterized in that**:
(a) a hydrocarbon compound which carries at least two functional groups, selected from primary amines, secondary amines, hydroxyl groups and thiol groups, is provided;
(b) a first one of the two functional groups is reacted with optionally activated (meth)acrylic acid and the second one of the two functional groups is reacted with a second carboxylic acid that is optionally activated and has a C=C double bond, and
(c) subsequent to the aforementioned reaction, a sulfonate group-containing compound or a sulfate group-containing compound or a sulfite is added to the C=C double bond of the carboxylic acid residue reacted with said second functional group in such a way that on the (meth)acryl residue reacted with said first functional group such an addition does not take place,
wherein the second C=C double bond-containing carboxylic acid is (meth)acrylic acid or another double bond-containing carboxylic acid.

8. A method for preparing a compound of the formula (II) as defined in claim 4, **characterized in that**:
(a) a hydrocarbon compound which carries at least one hetero ring, selected from oxacycyclopropyl, azacyclopropyl, thiocyclopropyl, and a cyclic carbonate, is provided,
(b) the hetero ring is reacted with a sulfite or a sulfonate group-containing compound or a sulfate group-containing compound, and
(c) at least the OH-, SH- or NH₂-group generated thereby is reacted with optionally activated (meth)acrylic acid.

9. The method according to claim 7 or 8, wherein the sulfonate group-containing compound or the sulfate group-containing compound has an OH-, SH- or NHR¹¹-group, with R¹¹ = hydrogen or C₁-C₆ alkyl, and in particular an SH group.

10. A method for preparing a compound of the formula (II) as defined in claim 4, **characterized in that**:
(a) an organic compound with at least three (meth)acryl groups is provided, and
(b) a sulfonate group-containing compound or a sulfate group-containing compound, containing an SH group, is added in less than stoichiometric quantity to the C=C double bond of one or several of the (meth)acryl groups such that the addition does not take place at least at one (meth)acryl residue.

11. A method for preparing a compound of the formula (II) as defined in claim 4, **characterized in that**:
(a) a (meth)acrylsilane with at least two (meth)acryl groups, which are bonded by a carbon atom of a hydrocarbon group to the silicon atom, is provided, and
(b) a sulfonate group-containing compound or a sulfate group-containing compound, containing an SH group, is added in less than stoichiometric quantity to the C=C double bond of one or several of the (meth)acryl residues such that the addition does not take place at least at one (meth)acryl residue.

12. A method for preparing a compound as defined in claim 6, **characterized in that** an organic compound, which has at least one first (meth)acryl residue and at least one alkenylcarboxyl residue, is reacted with a sulfonate group-containing compound or a sulfate group-containing compound or a sulfite so that the sulfonate group-containing compound or the sulfate group-containing compound or the sulfite is added to the C=C double bond of the alkenylcarboxyl residue in such a way that at the first (meth)acryl residue no such addition takes place, wherein the at least one alkenylcarboxyl residue can be a second (meth)acryl residue or the residue of another alkenylcarboxylic acid.

13. A silicic acid (hetero) polycondensate, **characterized in that** it is obtained by a hydrolytic condensation using a silane of the formula (I), (Ia) or (II).

14. The silicic acid (hetero) polycondensate according to claim 13, **characterized in that** it is obtained by additionally using at least one hydrolytically condensable metal compound of a metal, selected from metals of the main groups III and IV and metals of the transition metal groups III to VI.

15. The silicic acid (hetero) polycondensate according to one of claims 13 and 14, **characterized in that** it is water-soluble.

16. A polymerized product, obtained from or using at least one compound as defined in any one of claims 1 to 6 by polymerizing at least a portion of the (meth)acryl groups thereof, wherein optionally the polymerized product additionally contains an inorganically condensable network made of or with the use of Si-O-Si linkages.

17. The use of a polymerized product according to claim 16 or of a silicic acid (hetero)polycondensate according to any one of claims 13 to 15 for dental uses, in particular as a dental adhesive.

## Revendications

1. Composé, comprenant au moins trois fonctionnalités, à savoir (a) un groupe sulfonate ou sulfate de la formule - (O)_{d}-SO₃M avec d = 0 ou 1 et M = hydrogène ou un cation métallique monovalent ou la fraction correspondante d'un cation métallique polyvalent, (b) un radical (méth)acryle, ainsi que (c) soit (c1) une fonction acide carboxylique et/ou (c2) un groupe thioéther, avec la condition que dans un composé exempt de silicium un groupe sulfonate ou sulfate et un radical (méth)acrylate soient séparés l'un de l'autre par un radical hydrocarboné, dont la chaîne carbonée est interrompue par 0, S ou NH ou contient un groupe de liaison.

2. Composé selon la revendication 1, qui est un silane de la formule (I)
R⁷ₐR²_{b}SiZ_{4-a-b} (I),
dans laquelle R⁷ est un radical hydrolytiquement condensable, R² est un groupe alkyle, aryle, arylalkyle, alkylaryle ou alkylarylalkyle substitué ou non substitué, à chaîne droite, ramifié ou présentant un cycle ou est un groupe alcényle correspondant, dont la chaîne carbonée peut dans tous les cas être interrompue éventuellement par des groupes -0-, -S-, - NH-, -S(O)-, -C(O)NH-, -NHC(O)-, -C(O)O-, -C(O)S-, -NHC(O)NH- ou C(O)NHC(O), qui peuvent éventuellement être orientés dans les deux directions possibles, Z est un radical, dans lequel au moins un groupe (méth)acryle et au moins soit un groupe sulfonate soit un groupe sulfate se trouvent liés directement ou indirectement par un groupe hydrocarboné non substitué ou substitué à l'atome de silicium avec une liaison Si-C, a est 1, 2 ou 3, b est 0, 1 ou 2 et a+b valent ensemble 2 ou 3.

3. Silane selon la revendication 1 ou 2 avec la formule 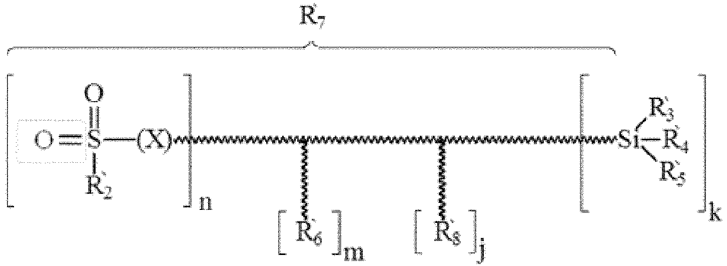 dans laquelle les groupes et les indices ont la signification suivante:
R'₂ représente OH ou un sel -OM avec M = cation métallique monovalent ou la fraction correspondante d'un cation métallique polyvalent, de préférence choisi parmi les cations alcalins et alcalino-terreux, en particulier parmi Na, K, 1/2Ca, 1/2 Mg ou ammonium,
X signifie oxygène ou n'est pas présent,
R'₃, R'₄, R'₅ sont soit des radicaux, qui peuvent être soumis à une condensation hydrolytique avec formation de ponts Si-0-Si soit des radicaux, qui sont liés à l'atome de silicium par un atome de carbone;
R'₆ est un radical à liaison double;
R'₇ désigne le radical organique, qui présente aussi bien le groupe sulf(on)ate que le radical à liaison double; celui-ci est lié au silicium par un atome de carbone,
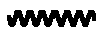 désigne un radical organique, qui peut être interrompu par au moins un groupe de liaison, choisi parmi les groupes - C(0)0-, -C(O)NH-, -NHC(O)-, -NHC(0)0-, -C(O)NHC(O)-, - NHC(0)NH-, -S(0)- ou les groupes sulfurés correspondants ou un atome d'oxygène, un atome de soufre ou un groupe aminé secondaire ou tertiaire,
R'₈ signifie -CO₂H ou OH,
n, m et j signifient respectivement au moins 1, mais peuvent dans certains cas être 2 ou 3 ou même prendre une valeur encore plus élevée.

4. Composé selon la revendication 1 avec la formule (II) 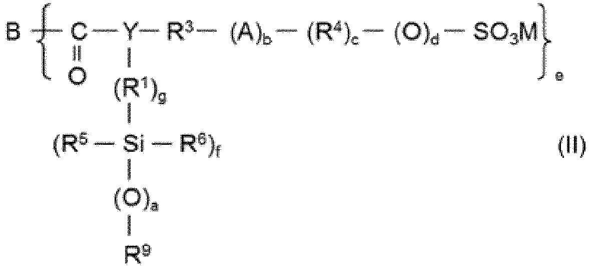 dans laquelle
R¹ est un radical hydrocarboné à liaison double, éventuellement interrompu par un ou plusieurs atomes d'oxygène, atomes de soufre, groupes ester, groupes amino ou groupes amide, qui, dans la mesure où f = 1, est lié à l'atome de silicium par un atome de carbone,
R⁹ est H ou un alkyle et de plus, dans le cas où a = 0, f = 1 et g = 1, peut être un radical hydrolytiquement condensable ou 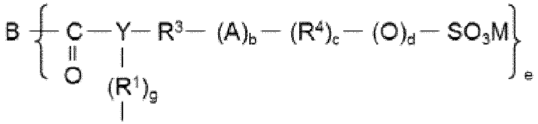
R³ est un alkylène non substitué ou substitué avec un groupe fonctionnel, à chaîne droite, ramifié ou présentant au moins un cycle,
A représente un groupe de liaison,
R⁴ signifie un groupe hydrocarboné éventuellement interrompu par 0, S, NH ou NR⁸ et/ou éventuellement substitué fonctionnellement, de préférence un tel alkylène,
M représente l'hydrogène ou un cation métallique monovalent ou la fraction correspondante d'un cation métallique polyvalent, de préférence choisi parmi les cations alcalins et alcalino-terreux, en particulier parmi Na, K, 1/2 Ca, 1/2Mg, ou ammonium,
R⁵ et R⁶ sont indépendamment l'un de l'autre soit des radicaux condensables dans des conditions d'hydrolyse soit un groupe alkyle, aryle, arylalkyle, alkylaryle ou alkylarylalkyle substitué ou non substitué, à chaîne droite, ramifié ou présentant au moins un cycle, mais qui peuvent à titre exceptionnel être aussi au lieu de ceux-ci un alcényle, arylalcényle ou alcénylaryle correspondant,
R⁸ est un alkyle ou un alcényle avec de préférence 1 à 6 ou 2 à 6 atomes de carbone ou est un radical (méth)acryle,
B est un vinyle, 2-allyle ou, dans le cas où e > 1, un radical organique avec e groupes vinyle, qui sont liés respectivement à un groupe se trouvant dans les accolades,
Y est un atome d'azote, -0-CH=, -S-CH=, ou -NH-CH=, dans lequel respectivement l'atome d'oxygène, l'atome de soufre ou le groupe NH présente une liaison avec le groupe C(0) voisin,
a = 0 ou 1,
b = 0 ou 1,
c = 0 ou 1,
d = 0 ou 1, et
e = 1, 2 ou 3,
f = 0 ou 1, et
g = 0 ou 1,
dans lequel, lorsque f = 1, a et g sont tous deux ≠ 0 et dans lequel, lorsque f = 0, au moins un des radicaux R³ ou R⁴ porte un substituant fonctionnel, qui présente un radical (méth)acryle ou un groupe acide ou R⁴ représente un alkylène interrompu au moins par S.

5. Composé selon l'une quelconque des revendications précédentes, dans lequel au moins un des radicaux R³ et R⁴ est substitué avec un radical R¹⁰COOM et éventuellement avec au moins un groupe hydroxy et/ou avec un radical SO₃M, dans lequel M est défini comme dans la revendication 1, dans lequel R¹⁰ est une liaison chimique ou un radical alkylène C₁-C₆.

6. Composé selon la revendication 4, dans lequel f = 0 et g = 0 et a = 0.

7. Procédé de fabrication d'un composé avec la formule (II) défini comme dans la revendication 4, **caractérisé en ce que**:
(a) on prépare un composé hydrocarboné, qui porte au moins deux groupes fonctionnels, choisis parmi les amines primaires, les amines secondaires, les groupes hydroxy et les groupes thiol,
(b) on fait réagir un premier des deux groupes fonctionnels avec l'acide (méth)acrylique éventuellement activé et le deuxième des deux groupes fonctionnels avec un deuxième acide carboxylique, éventuellement activé, présentant une liaison double C=C, et
(c) à la suite de la réaction précitée, on ajoute un composé contenant des groupes sulfonate ou sulfate ou un sulfite à la liaison double C=C du radical acide carboxylique ayant réagi avec le deuxième groupe fonctionnel, de telle manière qu'une telle addition ne se produise pas au radical (méth)acryle ayant réagi avec le premier de deux groupes fonctionnels,
dans lequel le deuxième acide carboxylique présentant une liaison double C=C peut être l'acide (méth)acrylique ou un autre acide carboxylique contenant une liaison double.

8. Procédé de fabrication d'un composé de la formule (II) défini comme dans la revendication 4, **caractérisé en ce que**
(a) on prépare un composé hydrocarboné, qui porte au moins un hétéro-anneau, choisi parmi oxacyclopropyle, azacyclopropyle, thiocyclopropyle et un carbonate cyclique,
(b) on fait réagir l'hétéro-anneau avec un sulfite ou un composé contenant des groupes sulfonate ou sulfate, et
(c) on fait réagir au moins le groupe OH-, SH- ou NH₂- ainsi libéré avec l'acide (méth)acrylique éventuellement activé.

9. Procédé selon une des revendications 7 ou 8, **caractérisé en ce que** le composé contenant des groupes sulfonate ou sulfate présente un groupe OH-, SH- ou NHR¹¹ avec R¹¹ = hydrogène ou un alkyle C₁-C₆, en particulier un groupe SH-.

10. Procédé de fabrication d'un composé de la formule (II) défini comme dans la revendication 4, **caractérisé en ce que**
(a) on prépare un composé organique avec au moins trois groupes (méth)acryle, et
(b) on ajoute un composé contenant des groupes sulfonate ou sulfate, qui présente un groupe SH-, par-dessous à la liaison double C=C d'un ou de plusieurs des radicaux (méth)acryle, de telle manière qu'une telle addition ne se produise pas à au moins un radical (méth)acryle dans la molécule.

11. Procédé de fabrication d'un composé de la formule (II) défini comme dans la revendication 4, **caractérisé en ce que**
(a) on prépare un (méth)acrylsilane avec au moins deux groupes (méth)acryle, qui sont liés à l'atome de silicium par un atome de carbone d'un groupe hydrocarboné, et
(b) on ajoute un composé contenant des groupes sulfonate ou sulfate, qui présente un groupe SH-, par-dessous à la liaison double C=C d'un ou de plusieurs des radicaux (méth)acryle, de telle manière qu'une telle addition ne se produise pas à au moins un radical (méth)acryle.

12. Procédé de fabrication d'un composé défini comme dans la revendication 6, **caractérisé en ce que** l'on fait réagir un composé organique, qui présente au moins un radical (méth)acryle et au moins un radical alcénylcarboxylique, avec un composé contenant des groupes sulfonate ou sulfate ou avec un sulfite, **en ce que** l'on ajoute celui-ci/ceux-ci à la liaison double C=C du radical alcénylcarboxylique, de telle manière qu'une telle addition ne se produise pas au radical (méth)acryle, dans lequel ledit au moins un radical alcénylcarboxylique peut être un deuxième radical (méth)acryle ou le radical d'un autre acide alcényl-carboxylique.

13. (Hétéro)polycondensat d'acide silicique, **caractérisé en ce qu'**il a été produit par condensation hydrolytique avec utilisation d'un silane de la formule (I), (Ia) ou (II).

14. (Hétéro)polycondensat d'acide silicique selon la revendication 13, **caractérisé en ce qu'**il a été produit avec utilisation supplémentaire d'au moins un composé métallique hydrolytiquement condensable d'un métal, choisi parmi les métaux du groupe III ou du groupe IV ainsi que des sous-groupes III à VI.

15. (Hétéro)polycondensat selon une des revendications 13 ou 14, **caractérisé en ce qu'**il est hydrosoluble.

16. Polymérisat, obtenu à partir de ou par utilisation d'un composé défini comme dans une des revendications 1 à 6 par polymérisation d'au moins une partie de leurs groupes (méth)acryle, dans lequel le polymérisat présente en plus en option un réseau inorganiquement condensé composé de ou avec des ponts Si-O-Si.

17. Utilisation d'un polymérisat selon la revendication 16 ou d'un (hétéro)polycondensat d'acide silicique selon l'une quelconque des revendications 13 à 15 pour des applications dentaires, en particulier pour un adhésif dentaire.
